# EUROPÄISCHE PATENTSCHRIFT

(11) **EP 0 743 304 B1**
(45) Veröffentlichungstag und Bekanntmachung des Hinweises auf die Patenterteilung: **26.03.2003**
(21) Anmeldenummer: 96107518.1
(22) Anmeldetag: 10.05.1996
(51) Int. Cl.: C07D 231/22, C07D 231/24, C07D 231/26, A61K 31/415

(54) **4-(Arylaminomethylen)-2,4-dihydro-pyrazol-3-one**
4-(Arylaminomethylene)-2,4-dihydro-pyrazol-3-ones
4-(Arylaminométhylène)-2,4-dihydro-pyrazole-3-ones

(30) Priorität: 17.05.1995 DE 19518082
(43) Veröffentlichungstag der Anmeldung: 20.11.1996
(73) Patentinhaber: MERCK PATENT GmbH, 64293 Darmstadt (DE)
(72) Erfinder: Arlt, Michael, Dr., 64342 seeheim (DE); Jonas, Rochus, Dr., 64291 Darmstadt (DE); Christadler, Maria, 63322 Rödermark (DE); Schneider, Günter, 64401 Gross Bieberau (DE); Klockow, Michael, Dr., 64380 Rossdorf (DE)

(56) Entgegenhaltungen:
- EP-A- 0 020 299
- EP-A- 0 201 188
- EP-A- 0 274 642
- WO-A-93/06104
- MONATSHEFTE FÜR CHEMIE, Bd. 112, 1981, Seiten 369-383, XP000579806 O.S. WOLFBEIS: "Eine effiziente Synthese von Aminoalkylidenderivaten fünfring- cyclischer methylenaktiver Verbindungen"
- ARZNEIMITTEL-FORSCHUNG, Bd. 19, Nr. 10, 1969, Seiten 1721-1723, XP000579808 D. NARDI ET AL.: "Pyrazoline-5-one and Pyrazolidine-3,5-dione Derivatives with Antiphlogistic and Analgesic Activity"
- JOURNAL FÜR PRAKTISCHE CHEMIE, Bd. 319, Nr. 6, 1977, Seiten 905-910, XP000196303 W. FREYER ET AL.: "Pyrazolonderivate. II. Synthese von tautomeriefähigen Enaminen einiger 3-substituierter 1-Phenyl-delta2- pyrazolinone-(5)"
- ZH. ORG. KHIM., Bd. 10, Nr. 10, Oktober 1974, Seiten 2210-2218, XP000196289 L.N. KURKOVSKAYA ET AL.: "Aminomethylen..."
- ZH. ORG. KHIM., Bd. 11, Nr. 8, August 1975, Seiten 1734-1739, XP000196288 L.N. KURKOVSKAYA ET AL.: "Aminomethylen..."
- ARCHIV DER PHARMAZIE, Bd. 319, Nr. 10, Oktober 1986, Seiten 865-871, XP000196299 A. KREUTZBERGER ET AL.: "Entzündungshemmende Wirkstoffe, 10. Mitt. 1-(4-Chlorphenyl)-2-pyrazolin-5-on- Derivate"

## Beschreibung

Die Erfindung betrifft Δ²Pyrazolinon-5-Derivate der allgemeinen Formel I: 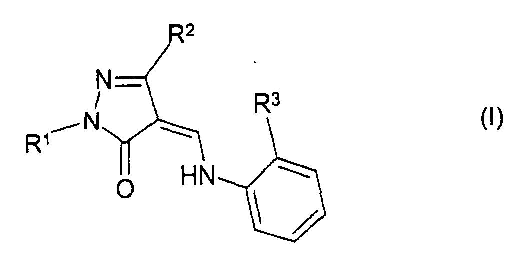 worin
- R¹: Benzyl, Alkoxybenzyl mit 1 - 3 C-Atomen im Alkylteil, Phenyl, ein- bis dreifach durch Amino, Acyl, Halogen, Nitro, CN, AO-, Carboxyl, Sulfonyl, A-O-CO-, A-CO-NH-, A-CO-NA-, Carbamoyl, N-Alkylcarbamoyl, N,N-Dialkylcarbamoyl (mit 1 - 6 C-Atomen im Alkylteil), A-O-CO-NH-, A-O-CO-NA-, A-SO₂-, SO₂NR⁴R⁵ (R⁴ und R⁵ H oder Alkyl mit 1 - 6 C-Atomen oder NR⁴R⁵ ein 5- oder 6-gliedriger Ring, wahlweise mit anderen Heteroatomen wie N, S, O, der durch A substituiert sein kann), A-CO-NH-SO₂-, A-CO-NA-SO₂-, A-SO₂-NH-, A-SO₂-NA-, (A-SO₂-)₂ N-, Tetrazolyl oder Phosphonyl-substituiertes Phenyl; oder Pyridyl,
- R²: Alkyl mit 1 - 5 C-Atomen, Alkoxycarbonylalkyl, Hydroxyalkyl, Hydroxycarbonylalkyl,
- R³: geradkettiges oder verzweigtes Alkyl mit 1 - 5 C-Atomen, geradkettiges oder verzweigtes Alkoxy mit 1 - 5 C-Atomen, durch Fluor oder Chlor substituiertes Alkyl,
- A: geradkettiges oder verzweigtes Alkyl mit 1 - 6 C-Atomen, geradkettiges oder verzweigtes, durch Fluor oder Chlor substituiertes Alkyl mit 1 - 6 C-Atomen
bedeuten,
sowie deren physiologisch unbedenklichen Salze,
wobei 5-Methyl-2-phenyl-4-(o-tolylamino-methylen)-2,4-dihydro-pyrazol-3-on ausgeschlossen ist.

Teil der Erfindung sind auch Verfahren zur Herstellung dieser Verbindungen, insbesondere aber deren Verwendung als selektive Inhibitoren der cGMP spezifischen Phosphodiesterase (cGMP PDE) und damit als pharmazeutisch wirksame Verbindungen.

Diese teilweise für andere Zwecke bekannten und als cGMP PDE-Inhibitoren wirksamen Verbindungen sind in verschiedenen Sektionen der Medizin therapeutisch einsetzbar. Insbesondere sind sie jedoch einsetzbar zur Behandlung von Krankheiten des Herz-Kreislaufsystems, der Herzinsuffizienz, der Arteriosklerose und anderen Beschwerden hervorgerufen durch Funktionsstörungen der Herzkranzgefäße.

Aus der Literatur ist eine Vielzahl von Verbindungen bekannt, die eine inhibierende Wirkung auf cGMP PD-Esterasen aufweisen.

So werden in EP-A1-0201 188 Pyrazolo[-4,3-d]pyrimidin-7-one als Adenosinrezeptor-Antagonisten und PDE-Inhibitoren beschrieben, die zur Behandlung von Krankheiten der Herzkranzgefäße bei Herzversagen oder Herzschwäche eingesetzt werden können. Jedoch sind in dieser Schrift weder Beispiele dieser Verbindungen gegeben, noch sind sie besonders wirksame PDE-Inhibitoren insbesondere für cGMP PDE.

In WO -A1-93/06104 werden substituierte Pyrazolo[-4,3]-pyrimidin-7-one mit einer gegenüber den in der erstgenannten Schrift offenbarten Verbindungsklasse verbesserten Inhibitorspezifität bezüglich cGMP PD-Esterasen im Vergleich zu der gegenüber cAMP PD-Esterasen. Die Selektivität dieser Verbindungen gegenüber den übrigen Phosphodiesterasen I, II und III bleibt jedoch in dieser Schrift unberücksichtigt.

In der EP 0 020 299 werden andere Pyrazolone beschrieben, die als Ausgangsmaterial für Nickelkomplexe von Azinen dienen.
Die Synthese weiterer Pyrazolone ist in Monatshefte für Chemie, 11, 369-383 (1981) beschrieben.
D. Nardi et al. beschreiben in Arzneimittel-Forschung 19 (10), 1721-23 (1969) weitere Pyrazolinone mit antiphlogistischer undanagetischer Wirkung.
Weitere Synthesen von Pyrazolinonderivaten findet man im Journal für praktische Chemie, 1977, 319 (6), 905-10, in Zh. Org. Khim., 10, 2210-2218 (1974) und in Zh. Org. Khim., 11, 1734-39(1975).
Andere Pyrazolinonderivate sind in Archiv der Pharmazie, 319, 865-871 (1986) beschrieben.

Die gleichzeitige inhibitorische Wirkung einer Verbindung auf die übrigen Phosphodiesterasen ist jedoch von großer Bedeutung, da sich bei gleichzeitiger Inhibitorwirkung auf weitere Esterasen außer auf cGMP-Phosphodiesterase (PDE V) ein ganzes Spektrum unerwünschter Nebenwirkungen bei ihrer Verwendung als Arzneimittel ergeben kann.

Es ist daher Aufgabe dieser Erfindung, Verbindungen zur Verfügung zu stellen, die eine besonders ausgeprägte Inhibitorwirkung auf cGMP-Phosphodiesterasen (PDE V) aufweisen, gleichzeitig jedoch auf die übrigen Phosphodiesterasen keine bzw. eine so geringe Inhibierung zeigen, daß Nebenwirkungen, die auf die Inhibierung der PD-Esterasen I - IV zurückzuführen sind, nicht nachweisbar sind.

Gleichzeitig ist es Aufgabe dieser Erfindung, ein Verfahren Verfügung zu stellen, wodurch die entsprechenden Verbindungen in möglichst hohen Ausbeuten und möglichst hohen Reinheiten hergestellt werden können.

Es wurde nun gefunden, daß Verbindungen der Formel (I) diese Aufgabe lösen.

Gegenstand der Erfindung sind weiterhin die neuen Verbindungen der allgemeinen Formel (I), worin
- R¹: Benzyl, Alkoxybenzyl mit 1 - 3 C-Atomen im Alkylteil, Phenyl, ein- bis dreifach durch Amino, Halogen, Nitro, Cyano, Carboxyl, A-O-CO-, A-CO-NH-, A-CO-NA-, Carbamoyl, N-Alkylcarbamoyl, N,N-Dialkylcarbamoyl (mit 1 - 6 C-Atomen im Alkylteil), A-SO₂-NH-, A-SO₂-NA, Aminoacyl, HSO₃ -, SO₂NR⁴R⁵ (R⁴ und R⁵ H oder Alkyl mit 1 - 6 C-Atomen oder NR⁴R⁵ ein 5- oder 6-gliedriger Ring, wahlweise mit anderen Heteroatomen wie N, S, O, der durch A sustituiert sein kann), A-CO-NH-SO₂-, A-CO-NH-SO₂- Tetrazolyl oder Phosphonyl substituiertes Phenyl; Pyridyl,
- R²: Hydroxyalkyl- und
- R³: geradkettiges oder verzweigtes Alkyl- mit 1 - 5 C-Atomen, geradkettiges oder verzweigtes Alkoxy- mit 1 - 5 C-Atomen, durch Fluor oder Chlor substituiertes Alkyl,
- A: geradkettiges oder verzweigtes Alkyl mit 1 - 6 C-Atomen, geradkettiges oder verzweigtes, durch Fluor oder Chlor substituiertes Alkyl mit 1 - 6 C-Atomen
bedeuten, oder deren Salze.

Insbesondere sind die Verbindungen
N-3(4-(2-Ethylanilinomethylen)-4,5-dihydro-3-methyl-5-oxo-1H-pyrazol-1-yl)-phenyl)-carbaminsäuremethylester
4-((2-Ethoxyanilinomethylen)-4,5-dihydro-3-methyl-5-oxo-1H-pyrazol-1-yl)-N-ethylbenzolsulfonamid
2-(1-4-(N,N-Diethylsulfamoyl)-phenyl)-4-((2-ethylanilino)-methylen)-4,5-dihydro-5-oxo-1H-pyrazol-3-yl)-essigsäureethylester
2-(1-4-(N,N-Diethylsulfamoyl)-phenyl)-4-((2-ethoxylanilino)-methylen)-4,5-dihydro-5-oxo-1H-pyrazol-3-yl)-essigsäureethylester
2-(1-(4-Acetamidophenyl)-4-((2-ethylanilino)-methylen)-4,5-dihydro-5-oxo-1H-pyrazol-3-yl)-essigsäureethylester
2-(4-((2-Ethylanilino)-methylen)-4,5-dihydro--5-oxo-1-(4-trifluoracetamidophenyl)-1H-pyrazol-3-yl)-essigsäureethylester
2-(1-(4-Ethoxycarbonylaminophenyl)-4-((2-ethylanilino)-methylen)-4,5-dihydro-5-oxo-1H-pyrazol-3-yl)-essigsäureethylester
2-(4-((2-Ethylanilinomethylen)-4,5-dihydro-1-(4-methansulfonamidophenyl)-5-oxo-1H-pyrazol-3-yl)-essigsäureethylester
N-(3-(4-(2-Ethylanilinomethylen)-4,5-dihyd ro-3-methyl-5-oxo-5H-pyrazol-1-yl)-phenyl)-acetamid
N,N-Diethyl-4-(4-(2-ethylanilinomethylen)-4,5-dihydro-3-methyl-5-oxo-1H-pyrazol-1-yl)-benzolsulfonamid
N-Ethyl-4-(4-(2-ethylanilinomethylen)-4,5-dihydro-3-methyl-5-oxo-1H-pyrazol-1-yl)-benzolsulfonamid
4-(2-Ethoxyanilinomethylen)-2,4-dihydro-5-methyl-2-(4-(4-morpholinylsulfonyl )-phenyl)-3H-pyrazol-3-on
4-(2-Ethylanilinomethylen)-2,4-dihydro-5-methyl-2-(4-(4-methyl-1-piperazinylsulfonyl)-phenyl)-3H-pyrazol-3-on
N-(3-(4-(2-Ethylanilinomethylen)-4,5-dihydro-3-methyl-5-oxo-1H-pyrazol-1-yl)-phenyl)-methansulfonamid
N-(3-(4-(2-Ethylanilinomethylen)-4,5-dihydro-3-methyl-5-oxo-1H-pyrazol-1-yl)-phenyl)-trifluoracetamid
N-(4-(4-(2-Ethylanilinomethylen)-4,5-dihydro-3-methyl-5-oxo-1H-pyrazol-1-yl)-phenyl)-N-methylsulfonylmethansulfonamid
N,N-Diethyl-4-(4,5-dihydro-4-(2-ethoxyanilinomethylen)-3-methyl-5-oxo-1H-pyrazol-1-yl)-benzolsulfonamid
N,N-Diethyl-4-(4,5-dihydro-4-(2-methoxyanilinomethylen)-3-methyl-5-oxo-1H-pyrazol-1-yl)-benzolsulfonamid
3-(4-(2-Ethylanilinomethylen)-4,5-dihydro-5-oxo-3-propyl-1H-pyrazol-1-yl)-benzolsulfonsäure
4-(4-(2-Ethylanilinomethylen)-4,5-dihydro-5-oxo-3-propyl-1H-pyrazol-1-yl)-benzolsulfonsäure
2-(4-(2-Ethylanilinomethylen)-4,5-dihydro-1-(4-nitrophenyl)-5-oxo-1H-pyrazol-3-yl)-essigsaeu reethylester
4-(4-(2-Ethylanilinomethylen)-4,5-dihydro-5-oxo-3-propyl-1H-pyrazol-1-yl)-benzoesäure
4-(4-(2-Ethylanilinomethylen)-4,5-dihydro-3-methyl-5-oxo-1H-pyrazol-1-yl)-N-hexylbenzamid
4-(4-(2-Ethylanilinomethylen)-4,5-dihydro-3-methyl-5-oxo-1H-pyrazol-1-yl)-benzamid
N,N-Diethyl-4-(4-(2-ethylanilinomethylen)-4,5-dihydro-3-methyl-5-oxo-1H-pyrazol-1-yl)-benzamid
4-(2-Ethylanilinomethylen)-2,4-dihydro-5-propyl-2-(4-pyridyl)-3H-pyrazol-3-on
N,N-Diethyl-4-(4-(2-ethylanilinomethylen)-4,5-dihydro-5-oxo-3-propyl-1H-pyrazol-1-yl)-benzamid
4-(4-(2-Ethylanilinomethylen)-4,5-dihydro-5-oxo-3-propyl-1H-pyrazol-1-yl)-N-hexylbenzamid
4-(4-(2-Ethylanilinomethylen)-4,5-dihydro-5-oxo-3-propyl-1H-pyrazol-1-yl)-benzamid
4-(2-Ethylanilinomethylen)-2,4-dihydro-5-propyl-2-(4-(1H-tetrazol-5-yl)-phenyl)-3H-pyrazol-3-on
4-(2-Ethylanilinomethylen)-2,4-dihydro-5-methyl-2-(3-(1H-tetrazol-5-yl)-phenyl)-3H-pyrazol-3-on
4-(4,5-Dihydro-3-methyl-5-oxo-4-(2-trifluormethylanilinomethylen)-1H-pyrazol-1-yl)-benzoesäure
4-(4-(2-Ethylanilinomethylen)-3-ethoxycarbonylmethyl-4,5-dihydro-5-oxo-1H-pyrazol-1-yl)-benzoesäure
4-(4,5-Dihydro-3-methyl-5-oxo-4-(2-(2-propinyloxy)-anilinomethylen)-1Hpyrazol-1-yl)-benzoesäure
4-(4,5-Dihydro-3-methyl-5-oxo-4-(2-propoxyanilinomethylen)-1H-pyrazol-1-yl)-benzoesäure
4-(4,5-Dihydro-4-(2-isopropylanilinomethylen)-3-methyl-5-oxo-1H-pyrazol-1-yl)-benzosäure
3-(4-(2-Ethylanilinomethylenaminomethylen)-4,5-dihydro-3-methyl-5-oxo-1H-pyrazol-1-yl)-benzolsulfonamid
2-(1-(4-Methoxycarbonylaminophenyl)-4-((2-ethylanilino)-methylen)-4,5-dihyd ro-5-oxo-1H-pyrazol-3-yl)-essigsäureethylester
2-(1-(4-(N,N-Diethylsulfamoyl)-phenyl)-4-(2-ethylanilinomethylen)-4,5-dihydro-5-oxo-1H-pyrazol-3-yl)-essigsäureethylester
2-(1-(4-(N,N-Diethylsulfamoyl)-phenyl)-4-(2-ethoxyanilinomethylen)-4,5-dihydro-5-oxo-1 H-pyrazol-3-yl)-essigsäureethylester
2-(1-(4-Acetamidophenyl)-4-(2-ethylanilinomethylen)-4,5-dihydro-5-oxo-1H-pyrazol-3-yl)-essigsäureethylester
2-(4-(2-Ethylanilinomethylen)-4,5-dihydro-5-oxo-1-(4-trifluoroacetamidophenyl)-1H-pyrazol-3-yl)-essigsäureethylester
2-(4-(2-Ethylanilinomethylen)-4,5-dihydro-1-(4-methoxycarbonylaminophenyl)-5-oxo-1H-pyrazol-3-yl)- essigsäureethylester
2-(4-(2-Ethylanilinomethylen)-4,5-dihydro-1-(4-methansulfonamidophenyl)-5-oxo-1H-pyrazol-3-yl)-essigsäureethylester
2-(1-(4-Acetamidophenyl)-4-(2-ethylanilinomethylen)-4,5-dihydro-5-oxo-1H-pyrazol-3-yl)-essigsäureethylester
2-(4-(2-Ethylanilinomethylen)-4,5-dihydro-1-(4-methoxycarbonylaminophenyl)-5-oxo-1H-pyrazol-3-yl)-essigsäure
N-(3-(4-(2-Ethylanilinomethylen)-4,5-dihydro-5-oxo-3-propyl-1H-pyrazol-1-yl)-phenyl)-methansulfonamid
N-(3-(4-(2-Ethylanilinomethylen)-4,5-dihydro-5-oxo-3-propyl-1H-pyrazol-1-yl)-phenyl)-acetamid
N-(3-(4-(2-Ethylanilinomethylen)-4,5-dihydro-5-oxo-3-propyl-1H-pyrazol-1-yl)-phenyl)-carbaminsäuremethylester
2-(4-(2-Ethylanilinomethylen)-4,5-dihydro-5-oxo-1-(3-trifluoroacetamidophenyl)-1H-pyrazol-1-yl)-essigsäureethylester
2-(4-(2-Ethylanilinomethylen)-4,5-dihydro-1-(3-methansulfonamidophenyl)-5-oxo-1H-pyrazol-3-yl)-essigsäureethylester
sowie ihre physiologisch unbedenklichen Salze Gegenstand der Erfindung.

Gegenstand der Erfindung sind insbesondere Arzneimittel der allgemeinen Formel (I) gemäß Anspruch 1, wobei 5-Methyl-2-phenyl-4-(o-tolylaminomethylen)-2,4-dihydro-pyrazol-3-on ausgeschlossen ist.

Ebenso stellt die Verwendung der Arzneimittel als selektive Inhibitoren der cGMP spezifischen Phosphodiesterasen einen Teil der Erfindung dar, wobei besondere Ausgestaltungen der Erfindung die Verwendung der Verbindungen der allgemeinen Formel (I) und/oder der entsprechenden physiologisch unbedenklichen Salze oder der oben genannten Arzneimittel zur Herstellung von Arzneimittelformulierungen zur Behandlung von Krankheiten, insbesondere des Herz-Kreislaufsystems sowie der Herzinsuffizienz, sowie pharmazeutische Zubereitungen, die mindestens eine der oben genannten Verbindungen der Formel (I) und/oder mindestens eines ihrer physiologischen Salze enthalten oder der entsprechenden Arzneimittel, darstellen.

Aber auch pharmazeutische Zubereitungen, dadurch gekennzeichnet, daß sie mindestens eine Verbindung der oben gegebenen Formel (I), worin R¹, R² und R³ die oben gegebenen Bedeutungen haben, und/oder mindestens eines ihrer physiologischen Salze oder der entsprechenden als Inhibitoren wirkenden Arzneimittel enthalten und gemeinsam mit mindestens einem festen, flüssigen oder halbflüssigen Träger- oder Hilfsstoff in einer geeigneten Dosierungsform vorliegen, sind Gegenstand dieser Erfindung.

Ebenso stellt einen Teil der Erfindung ein Verfahren zur Herstellung der Verbindungen gemäß der oben gegebenen Formel (I) mit den oben definierten Substituenten R¹, R² und R³ oder der entsprechenden Inhibitoren dar, dadurch gekennzeichnet, daß Verbindungen allgemeinen Formel II 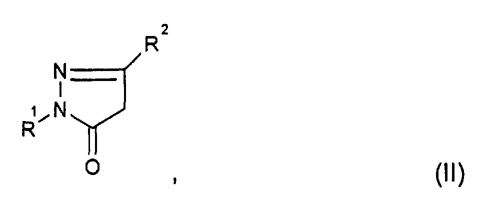 worin R¹ und R² die oben angegebenen Bedeutungen haben, mit geeigneten Formaldehyd-spendenden Verbindungen, wie Triazin oder geeigneten Trialkylorthoformiaten, inbesondere Trimethylorthoformiat umgesetzt zu Verbindungen der allgemeinen Formel 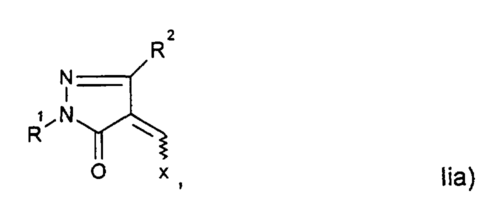 worin X eine Amino- oder eine -O-Alkyl-Gruppe (mit 1-6 C-Atomen im Alkyl) bedeutet, umgesetzt werden und diese, gegebenenfalls in situ, mit geeigneten Anilinderivaten der Formel III 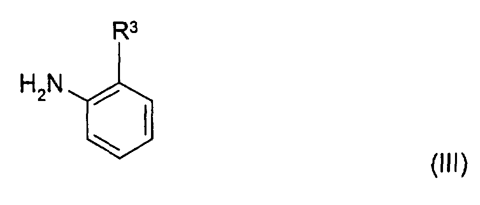 worin R³ die gegebenen Bedeutungen besitzen kann, oder deren Salze, gegebenenfalls in geeigneten Lösungsmitteln, zu Verbindungen der Formel I umgesetzt werden,
und/oder daß man in einer Verbindung der Formel I einen oder mehrere Rest(e) R in einen oder mehrere andere Reste R umwandelt.

Verbindungen der Formel (I) mit zum größten Teil anderen Substituenten sind aus der Anmeldung EP-B1-0 274 642 als herbizide und fungizide Mittel bekannt. Daher war es überraschend, zu finden, daß Verbindungen der Formel I gleichfalls als selektive Inhibitoren der c-GMP spezifischen Phosphodiesterase wirken und u. a. zur Behandlung von Krankheiten des Herz-Kreislaufsystems und der Herzinsuffizienz verwendet werden können.

Besonders vorteilhaft bei der Verwendung erfindungsgemäßer Verbindungen als pharmazeutisch wirksame Substanzen ist die sehr spezifische Inhibierung der cGMP-Phosphodiesterasen (PDE V), während für die Phosphodiesterasen PDE I, II, III und IV eine mehr als zehntausendfach geringere, d. h. keine nennenswerte, Inhibierung gemessen werden kann. Dementsprechend treten bei der Verwendung derart spezifisch wirkender Verbindungen als Arzneimittel keine Nebenwirkungen auf, die üblicherweise durch die Inhibierung der übrigen Phosphodiesterasen entstehen.

Verbindungen der Formel II, sowie auch deren Ausgangsstoffe können nach dem Fachmann aus zahlreichen Veröffentlichungen bekannten oder nach leicht abgewandelten Methoden hergestellt werden. Entsprechende Methoden sind auch in der Patentschrift EP-B1-0 274 642 oder in den Standardwerken wie Houben-Weyl, "Methoden der organischen Chemie", Georg-Thieme-Verlag, Stuttgart, beschrieben, bzw. aus der in dem Übersichtshandbuch "Pyrazolones, Pyrazolidones, and Derivatives", Wiley, R. H., Wiley, P.; Interscience Publishers, John Wiley & Sons (1964) angegebenen Sekundärliteratur bekannt oder in den folgenden Artikeln Ringel, C., Mayer, R., J. Prakt. Chem. **26** (1964) 333 ff; Gillespie, J. F.; Price, C. C., J. Org. Chem. **22** (1957) 780 ff;.Tabel, K., Kawashima, E., Kato, T., Chem. Pharm. Bull (CPBTAL), **29** (1) (1981), 244 ff ;Wilson, J. D., Fulmer, T. D., Dasher, L. P., Beam, C. F., J. Heterocycl. Chem. **17** (2) (1980) 389-391); Neunhoefer, H., Koehler, G., Degen H.-J.; Liebigs Ann. Chem. (1985),N 1 , 78-89 Ege, S., Adams, A. D., Gess, E. J., Ragone, K.

S., Kober, B. J., J. chem. Soc. Perkin Trans. (1983) N 2, 325-321;Pathak, R. B., Bahel, S. C., J. Indian Chem. Soc. **57** (1980) 1108-1111 ;Ali, M. I., El-Morsy, M. M. S., Hammouda H. A., Sharaf, M. F, Egypt. J. Chem. **22** (1979) 179-188; Mcevoy F. J., Albright J. D., J. org. Chem. **44** (1979) 4597-4603 beschrieben

In nachfolgenden Reaktionsschritten werden Verbindungen der Formel II weiter zu den erfindungsgemäßen Verbindungen der Formel I, die als geometrische Isomere oder Isomerengemische unterschiedlicher Zusammensetzung vorliegen können, umgesetzt. Dieses kann entweder über eine Zwischenstufe, bei der die 4-Position des Pyrazolinrings durch eine Methylengruppierung substituiert wird, und anschließende Umsetzung mit einem Anilinderivat erfolgen, oder die Substitution kann direkt durch ein entsprechendes Anilinderivat erfolgen. Die Wahl der Herstellungsvariante wird hierbei von den chemischen Eigenschaften der Substituenten des Pyrazolinons beeinflußt.

Einige der hergestellten Verbindungen der Formel I können im tautomeren Gleichgewicht vorliegen:

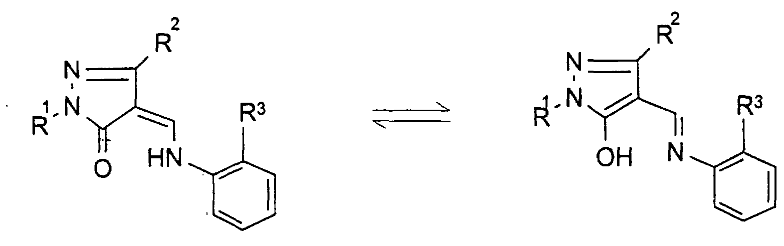

Es wird jedoch stets von der Verwendung der Verbindungen der Formel I gesprochen, obwohl sowohl die reinen Verbindungen als auch ihre Gemische mit unterschiedlichen Anteilen der tautomeren bzw. isomeren Verbindungen gemeint sind.

Insbesondere lassen sich sowohl die neuen als auch die bereits bekannten Verbindungen der Formel I durch Umsetzung von Verbindungen der allgemeinen Formel IIa 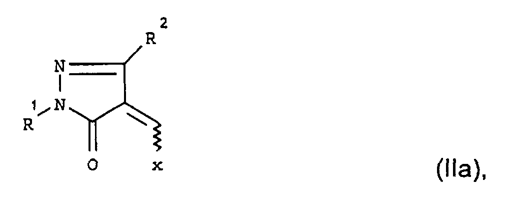 worin R¹ und R² die oben angegebenen Bedeutungen haben und X eine Amino- oder eine Alkoxygruppe bedeuten kann, mit geeigneten Anilinderivaten der Formel III 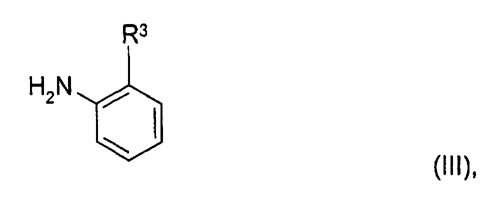 worin R³ die oben angegebenen Bedeutungen haben kann, herstellen.

Diese Umsetzung wird gegebenenfalls in Gegenwart eines geeigneten Verdünnungsmittels bei Temperaturen von 0 bis 120° C, insbesondere bei erhöhten Temperaturen durchgeführt.

Verbindungen der Formel lla lassen sich durch Umsetzung von geeigneten Verbindungen der allgemeinen Formel II 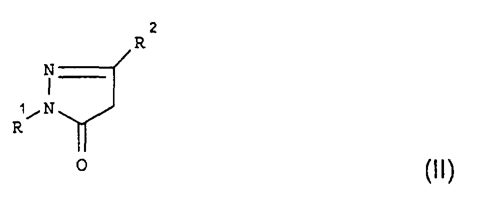 mit Formaldehyd-spendenden Gruppen aus der Gruppe Triazin, Dimethylformamid, Dimethylformamiddimethylacetal, Gold's Reagenz, Ameisensäurechlorid, Formamid oder Alkylderivate des Formamids mit 1-6 C-Atomen im Alkyl oder solcher aus der Gruppe der Trialkylorthoformiate, insbesondere Trimethylorthoformiat, herstellen. Gegebenenfalls erfolgt die Umsetzung in einem geeigneten, in der späteren Verwendung nicht störenden Verdünnungsmittel, wie beispielsweise Eisessig, und evtl. eines geeigneten Katalysators. Die Verbindungen der Formel lla können als Zwischenprodukte isoliert werden. Sie können aber auch direkt durch Umsetzung in situ mit entsprechenden Aminen der Formel III zu Verbindungen der Formel I weiter umgesetzt werden.

Ein anderer Syntheseweg zur Herstellung von Verbindungen der Formel I wird insbesondere dann bevorzugt, wenn die Verbindungen der Formel II empfindliche Substituenten besitzen, die in der Reaktion mit Formaldehyd-spendenden Verbindungen oder in der Umsetzung mit Anilinderivaten der Formel III bevorzugt reagieren. In diesen Fällen wird vorzugsweise ein entsprechendes Aryl-Isocyanat in bekannter Weise in Gegenwart einer Base, insbesondere von Butyl- oder Methyllithium, mit der entsprechenden Verbindung der Formel II umgesetzt.

Verbindungen der allgemeinen Formel II werden üblicherweise durch Umsetzung von β-Ketoestern bzw. 1,3-Dicarbonylverbindungen der allgemeinen Formel IV 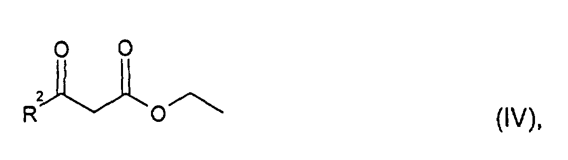 worin R² die oben angegebenen Bedeutungen haben kann, mit Hydrazinen der allgemeinen Formel V 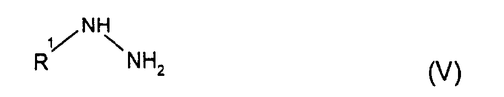 bzw. deren Salze, wie z. B. deren Hydrochloride, Hydrosulfate, Hydrooxalate u. a., gegebenenfalls in Gegenwart eines geeigneten Verdünnungsmittels, das sich in der weiteren Verwendung des Reaktionsprodukts nicht als störend erweist, wie beispielsweise Ethanol, und gegebenenfalls in Gegenwart eines geeigneten Katalysators, wie Toluolsulfonsäure, bei Temperaturen zwischen 0 und 120° C hergestellt.

Die verwendeten 1,3-Dicarbonylverbindungen der Formel IV sind allgemein bekannte Verbindungen der organischen Chemie und sind entweder im Handel erhältlich oder sind nach dem Fachmann allgemein bekannten Methoden zu synthetisieren.

Die zur Durchführung der Cyclisierungsreaktion benötigten Hydrazine sind bekannte Verbindungen oder können nach dem Fachmann allgemein bekannten Methoden erhalten werden (siehe z. B. Houben-Weyl, Methoden der organischen Chemie, Bd. X, 2, S. 203, Thieme Verlag Stuttgart 1967).

Wie bereits oben angesprochen, besitzen Verbindungen der vorliegenden Erfindung eine überdurchschnittliche Selektivität als Inhibitoren für cGMP PD-Esterasen. Unter Einfluß dieser Inhibitoren wird daher die cGMP-Konzentration im Organismus erhöht. Dieses wirkt sich in einer vorteilhaften Zunahme der Hemmung der Blutplättchenaggregation, sowie einer Zunahme der Granulozytenaktivität, des Vasospasmus und einer zunehmenden vasodilatorischen Aktivität ebenso wie durch eine Potentierung des Effekts des vom Endothelium abgeleiteten Entspannungsfaktors. Dementsprechend sind die Verbindungen einsetzbar zur Behandlung von verschiedenen Krankheiten, einschließlich der Hypertonie verschiedener Ausprägung, des Herzversagens verschiedener Ursachen, der Arteriesklerose, Folgen verengter Blutgefäße, beispielsweise bei Schlaganfall, Bronchitis, chronischem und allergischem Asthma, allergischem Schnupfen, Glaukom und Krankheiten, charakterisiert durch Störungen der Peristaltik der Verdauungsorgane.

Die biologischen Aktivitäten der Verbindungen gemäß der vorliegenden Erfindung wurden nach Methoden bestimmt, wie sie beispielsweise in der internationalen Anmeldung WO-A1-93/06104 beschrieben sind.

So wurden die Affinitäten der Verbindungen für cGMP und cAMP Phosphodiesterasen bestimmt durch die Ermittlung ihrer IC₅₀-Werte (Konzentration des Inhibitors, die benötigt wird, um eine 50%ige Inhibierung der Enzymaktivität zu erzielen). Zur Durchführung der Bestimmungen wurden nach bekannten Methoden isolierte Enzyme verwendet (z. B. nach: W. J. Thompson et. al.;Biochem, 1971, 10, 311). Zur Durchführung der Versuche wurde eine modifizierte "batch"-Methode von W. J. Thompson und M. M. Appleman (Biochem.,1979, 18, 5228) angewendet.

Ergebnisse dieser Versuche zeigen, daß die Verbindungen gemäß der allgemeinen Formel I wirksame und selektive Inhibitoren für cGMP Phosphodiesterasen sind. Dies gilt insbesondere für solche Verbindungen gemäß der allgemeinen Formel I, in denen R² ein Methyl-, Propyl-, Hydroxycarbonylmethyl oder Alkoxycarbonylmethyl-Rest und R¹ Benzoesäure, Benzolsulfonsäure, N-Methyl- bzw. N,N'-Dialkylbenzosulfonamid, Acylaminophenyl, N,N-Diethylbenzamid oder Benzoesäureamide bedeuten.

Verbindungen gemäß der allgemeinen Formel I, mit den Substituenten Methyl- oder Propyl- als R² und Benzoesäure, Benzamid, N-Hexyl-benzamid oder N,N-Diethylbenzamid, Benzolsulfonamid oder Acylaminophenyl als R¹ weisen eine besonders ausgeprägte Thrombozyten-aggregationshemmende Wirkung auf.

Die Verbindungen der allgemeinen Formel I und ihre physiologisch unbedenklichen Salze können daher zur Herstellung pharmazeutischer Präparate verwendet werden, indem man sie zusammen mit mindestens einem Träger- oder Hilfsstoff und, falls erwünscht, mit einem oder mehreren weiteren Wirkstoffen in die geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z. B. orale oder rektale) oder parenterale Applikation oder für die Applikation in Form eines Inhalationssprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk oder Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässrige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden. Die erfindungsgemäß beanspruchten Wirkstoffe können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine, Diuretika, Antiphlogistika.

Die erfindungsgemäßen Verbindungen gemäß Formel I werden in der Regel in Analogie zu anderen bekannten, im Handel erhältlichen Präparaten, insbesondere aber in Analogie zu den in der US-PS-4 880 804 beschriebenen Verbindungen verabreicht, vorzugsweise in Dosierungen zwischen etwa 1 mg und 1 g, insbesondere zwischen 50 und 500 mg pro Dosierungseinheit. Die tägliche Dosierung liegt vorzugsweise zwischen etwa 0.1 und 50 mg/kg, insbesondere 1 und 10 mg/kg Körpergewicht. Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Im folgenden werden Beispiele gegebenen, die zur Veranschaulichung der Erfindung dienen, jedoch die Erfindung nicht auf die gegebenen Beispiele begrenzen.

In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.

Vor- und nachstehend sind alle Temperaturen in ° C angegeben.

### Beispiele

### 1 a) 5 -Methyl -2 - (4-nitrophenyl)-2,4-dihydro -Dyrazol -3 -on

### (Cyclisierungsreaktion)

1.63 g p-Nitrophenylhydrazin-Hydrochlorid und 1.26 g Ethylacetoacetat werden in 30 ml Ethanol 45 min unter Rückfluß erhitzt. Die Mischung wird im Vakuum etwas eingeengt. Anschließend werden die ausgefallenen Kristalle abgesaugt.
- Ausbeute:: 1.20 g 5-Methyl-2-(4-nitrophenyl)-2,4-dihydro-pyrazol-3-on (64% der Theorie)
- Schmelzpunkt:: 223° C

### 1b) 4-(2-Ethylphenylaminomethylen)-5-methyl-2-(4-nitrophenyl)-2,4-dihydro-pyrazol-3-on

### (Einstufenreaktion: Formamid- und Anilinaddition)

1 g 5-Methyl-2-(4-nitrophenyl)-2,4-dihydro-pyrazol-3-on, 190 mg 1,3,5-Triazin und 0.74 ml 2-Ethylanillin werden in 50 ml Ethanol 4 Tage unter Rückfluß erhitzt. Das Lösungsmittel wird unter Vakuum abgedampft und das so erhaltene Rohprodukt der Reaktion wird chromatographisch an Kieselgel mit einem Lösungsmittelgemisch, bestehend aus Dichlormethan und Methanol im Mischungsverhältnis von 97:3, als Elutionsmittel gereinigt.
- Ausbeute:: 1.33 g 4-(2-Ethylphenylaminomethylen)-5-methyl-2-(4-nitrophenyl)- 2,4-dihydro-pyrazol-3-on (83% der Theorie)
- Schmelzpunkt:: 220° C.

### 1c) 3-Methyl-4-aminomethylen-1-phenyl-4,5-dihydro-pyrazol-5 -on

### (Formamidaddition)

Zu einer Suspension 52,3 g (0.3 mol) 3-Methyl-1-phenyl-2-pyrazolin-5-on in 800 ml Ethanol werden unter Rühren 8,11 g (0.1 mol) 1,3,5-Triazin zugefügt und unter Rückfluß eine Stunde gekocht. Anschließend wird die Lösung auf ein kleineres Volumen einrotiert. Die dabei ausgefallenen Kristalle werden in der Kälte abgesaugt. Auf diese Weise werden 24,5 g Kristalle erhalten, aus denen das 3- Methyl-4-aminomethylen-1-phenyl-4,5-dihydro -pyrazol-5-on chromatographisch an Silikagel mit einem Lösungsmittelgemisch aus Dichlormethan/Aceton im Verhältnis 4:1 als Elutionsmittel abgetrennt wird.

Durch Einrotieren der Mutterlauge werden 36 g eines Harzes erhalten, aus dem in gleicher Weise chromatographisch 14,1 g der dimeren Verbindung (FP: 180,6 ° C) abgetrennt werden. Es wird aus Aceton umkristallisiert.

### 1d) 3-Methyl-4- (2-propoxyphenylaminomethylen)-1-phenyl-4,5-dihydropyrazol-5-on

### (Anilinaddition)

2 g 3-Methyl-4-aminomethylen-1-phenyl-4,5-dihydro -pyrazol-5-on und 1,6 g 2-Propoxyanilino-trifluoressigsäuresalz werden in Ethanol gegeben und 1,5 Stunden unter Rückfluß gekocht. Die Reaktionslösung wird eingeengt. Anschließend wird das Reaktionsprodukt an Kieselgel mit einem Lösungsmittelgemisch aus Methylbutylketon/Hexan 4:1 als Elutionsmittel chromatographisch abgetrennt und aus einem Methylbutylketon/Hexan-Gemisch umkristallisiert.
- Ausbeute:: 1,5 g 3-Methyl-4-(2-propoxyphenylaminomethylen)-1-phenyl-4,5-dihydro -pyrazol-5-on (45,5 % der Theorie)
Es kann kein dimeres Produkt nachgewiesen werden.

### 1e) 4-(2-Methoxyphenylaminomethylen)-5-methyl-2-phenyl-2,4-dihydropyrazol-3-on

2 g 5-Methyl-2-phenyl-2,4-dihydro-pyrazol-3-on, 188 ml Trimethylorthoformiat und 1.29 ml o-Anisidin werden mit 5 ml Eisessig auf 70° C für 2 h unter Rühren erhitzt. Das Reaktionsgemisch wird abgekühlt und mit 10 ml Methanol versetzt. Der ausgefallene Niederschlag wird abgesaugt und aus Ethylacetat umkristallisiert.
- Ausbeute:: 1.1g 4-(2-Methoxyphenylaminomethylen)-5-methyl-2-phenyl-2,4-dihydro-pyrazol-3-on (31 % der Theorie)
- Schmelzpunkt:: 143° C

### 2a) 4-(4,5-Dihydro-4-(2-ethylanilinomethylen)3-methyl-5-oxo-1H-pyrazol-1-yl)-N-hexyl-benzamid

### (Nachträgliche Derivatisierung des 1-Substituenten)

0,5 g (1,43 mmol) 4-(4,5-Dihydro-4-(2-ethylanilinomethylen)-3-methyl-5-oxo-1H-pyrazol-1-yl)- benzoesäure und 0,19 ml (1,43 mmol) Hexylamin und 20 ml DMF werden bei Raumtemperatur in einem Reaktionskolben miteinander vermischt und etwa 5 Minuten gerührt. Nacheinander werden anschließend 0,27g (1,43 mmol) N-(3-Dimethylaminopropyl)-N'-ethylcarbodiimid·HCl, 0,19g (1,43 mmol) 1-Hydroxy-benzotriazol und 0,18 ml (1,43 mmol) N-Methylmorpholin hinzugefügt und 3 Stunden bei Raumtemperatur gerührt. Das Reaktionsende wird dünnschichtchromatographisch (DC in CH₂Cl₂/MeOH 9:1 (Ninhydrin-Sprühreagenz)) ermittelt.
Das Reaktionsgemisch wird anschließend in 200 ml Wasser aufgenommen (kein Niederschlag), zweimal mit Ethylether extrahiert, die vereinigten Etherphasen über Natriumsulfat getrocknet, anschließend filtriert, der Ether unter Vakuum abdestilliert und der so erhaltene Rückstand chromatographisch aufgearbeitet (Säule: Kieselgel Si₆O, Eluent: Methylbutylether).
- Ausbeute:: 250 mg 4-(4,5-Dihydro-4-(2-ethylanilinomethylen)-3-methyl-5-oxo-1H-pyrazol-1-yl)-N-hexyl-benzamid (41,6% der Theorie)

### 2b) 4 - (2-Ethylanilinomethylen)-4,5-dihydro-3-methyl-1-(3-(1H-tetrazol-5-yl)-phenyl)-1H-pyrazol-5-on

### (Nachträgliche Derivatisierung des 1-Substituenten)

200 mg (0,6 mmol) 3-(4,5-Dihydro-4-(2-ethylanilinomethylen)-3-methyl-5-oxo-1H-pyrazol-1-yl) -benzonitril, 480 mg (2,4 mmol) Trimethylzinnazid und 20 ml Toluol werden miteinander vermischt und unter Rühren zwei Tage unter Rückfluß erhitzt. Nach dieser Reaktionszeit sind noch geringe Mengen der Edukte chromatographisch nachweisbar. Die Aufarbeitung wird folgendermaßen durchgeführt:
Der Niederschlag, der sich während der Reaktion gebildet hat wird abgesaugt. Es handelt sich dabei um Produkt, das nur durch Sn-Salze verunreinigt ist. Das Produkt wird daher chromatigraphisch gereinigt (Säule: Kieselgel Si₆O, Eluent: CH₂Cl₂/MeOH 9:1).
- Ausbeute:: 100 mg 4-(2-Ethylanilinomethylen)-4,5-dihydro-3-methyl-1-(3-(1H-tetrazol-5-yl)-phenyl)-1H-pyrazol-5-on (45,5 % der Theorie)

Im einzelnen wurden außer den in den Herstellungsbeispielen 1 und 2 genannten Verbindungen folgende Pyrazol-3-on-Derivate der allgemeinen Formel I nach den beschriebenen Verfahren unter Verwendung von Triazin oder Trimethylorthoformiat hergestellt:
3. aus 4-(4-Morpholinyl)sulfonylphenylhydrazin und Ethylacetoacetat
   5-Methyl -2-(4-(4-morpholinyl)-sulfonylphenyl)-2,4-dihydro-pyrazol-3-on
   und 2-Ethylanilin
   4-(2-Ethylanilinomethylen)-2,4-dihydro-5-methyl-2-(4-(4-morpholinyl)-sulfonylphenyl)-3H-pyrazol-3-on, FP: 275° C
4. aus Phenylhydrazin-3-acetamid und Ethylacetoacetat
   N-(3-(-4,5-dihydro-3-methyl-5-oxo-5H-pyrazol-1-yl)-phenyl)-acetamid)
   und 2-Ethylanilin
   N-(3-(4-(2-Ethylanilinomethylen)-4,5-dihydro-3-methyl-5-oxo-5Hpyrazol-1-yl)-phenyl)-acetamid, FP: 263° C
5. aus N,N-Diethyl-4-hydrazinobenzolsulfonamid und Ethylacetoacetat
   N,N-Diethyl-4-(4,5-dihydro-3-methyl-5-oxo-1H-pyrazol-1-yl)-benzolsulfonamid
   und 2-Ethylanilin
   N,N-Diethyl-4-(4-(2-ethylanilinomethylen)-4,5-dihydro-3-methyl-5-oxo-1H-pyrazol-1-yl)-benzolsulfonamid, FP: 194 ° C
6. aus N-Ethyl-4-hydrazinobenzolsulfonamid und Ethylacetoacetat
   N-Ethyl-4-(4,5-dihydro-3-methyl-5-oxo-1H-pyrazol-1-yl)-benzolsulfonamid
   und 2-Ethylanilin
   N-Ethyl-4-(4-(2-ethylanilinomethylen)-4,5-dihydro-3-methyl-5-oxo-1H-pyrazol-1-yl)-benzolsulfonamid, FP: 260° C
7. aus 4-(4-Morpholinyl)-sulfonylphenylhydrazin und Ethylacetoacetat
   5-Methyl-2-(4-(4-morpholinyl)-sulfonylphenyl)-2,4-dihydro-pyrazol-3-on
   und 2-Butoxyanilin
   4-(2-Butoxyanilinomethylen)-2,4-dihydro-5-methyl-2-(4-(4-morpholinylsulfonyl)-phenyl)-3H-pyrazol-3-on, FP: 171° C
8. aus 4-(4-Morpholinyl)sulfonylphenylhydrazin und Ethylacetoacetat
   5-Methyl -2-(4-(4-morpholinyl)-sulfonylphenyl)-2,4-dihydro-pyrazol-3-on
   und 2-Ethoxyanilin
   4-(2-Ethoxyanilinomethylen)-2,4-dihydro-5-methyl-2-(4-(4-morpholinylsulfonyl)-phenyl)-3H-pyrazol-3-on, FP: 265° C
9. aus 4-(4-Methylpiperazinyl)-sulfonylphenylhydrazin und Ethylacetoacetat
   5-Methyl -2-(4-(4-methylpiperazinyl)-sulfonylphenyl)-2,4-dihydropyrazol-3-on
   und 2-Ethylanilin
   4-(2-Ethylanilinomethylen)-2,4-dihydro-5-methyl-2-(4-(4-methyl-1-piperazinylsulfonyl)-phenyl)-3H-pyrazol-3-on, FP: 254° C

### 10.a) 2 - (3-Aminophenyl)-5 -methyl-2,4-dihydropyrazol -3 -on

### (Hydrierung eines Substituenten)

Eine Lösung bestehend aus 15 g 5-Methyl-2-(3-nitrophenyl)-2,4-dihydro-pyrazol-3-on in 400 ml Methanol wird in Gegenwart von 10 g Raney-Nickel hydriert. Der Katalysator wird abfiltriert und der nach dem Einengen der Lösung im Vakuum erhaltene Rückstand wird aus Isopropanol umkristallisiert.
- Ausbeute:: 8.0 g 2-(3-Aminophenyl)-5-methyl-2,4-dihydro-pyrazol-3-on (62 % der Theorie)
- Schmelzpunkt:: 265° C.

Analog erhält man aus den entsprechenden Nitroverbindungen:
2-(4-Aminophenyl)-5-methyl-2,4-dihydro-pyrazol-3-on (amorph)
2-(2-Aminophenyl)-5-methyl-2,4-dihydro-pyrazol-3-on (amorph)

### 10b) Reaktion der Aminogruppe des N-Substituenten des Pyrazols

Eine Lösung von 4.0 g 2-(3-Aminophenyl)-5-methyl-2,4-dihydropyrazol-3-on in 30 ml Dichlormethan und 2 ml Pyridin wird unter Eiskühlung und Rühren mit 2.2 ml Methansulfonylchlorid versetzt und 2 Stunden nachgerührt. Anschließend wird die Lösung mit verdünnter Salzsäure und Wasser gewaschen, getrocknet und im Vakuum eingeengt.
Aus dem erhaltenen N-(3-(4,5-dihydro-3-methyl-5-oxo-1H-pyrazol-1-yl)-phenyl)-methansulfonamid
und 2-Ethylanilin erhält man
N-(3-(4-(2-Ethylanilinomethylen)-4,5-dihydro-3-methyl-5-oxo-1H-pyrazol-1-yl)-phenyl)-methansulfonamid, FP: 192° C

### 11. Reaktion der Aminogruppe des N-Substituenten des Pyrazols mit Chlorameisensäuremethylester bzw Methansulfonsäurechlorid

Eine Lösung von 4.0 g 2-(3-Aminophenyl)-5-methyl-2,4-dihydropyrazol-3-on in 30 ml Dichlormethan und 2 ml Pyridin wird unter Eiskühlung und Rühren mit 2.2 ml Chlorameisensäuremethylester versetzt und 2 Stunden gerührt. Anschließend wird die Lösung mit verdünnter Salzsäure und Wasser gewaschen, getrocknet und im Vakuum eingeengt
Ausbeute: 4.2 g N-(3-(4,5-dihydro-3-methyl-5-oxo-1H-pyrazol-1-yl)-phenyl)-carbaminsäuremethylester (76 % der Theorie), Öl

Durch weitere Umsetzung mit 2-Ethylanilin erhält man daraus N-(3(4-(2-Ethylanilinomethylen)-4,5-dihydro-3-methyl-5-oxo-1H-pyrazol-1-yl)-phenyl-carbaminsäuremethylester, FP. 229 °C

Analog erhält man aus
2-(4-Aminophenyl)-5-methyl-2,4-dihydro-pyrazol-3-on und Methansulfonsäurechlorid
N-(4-(4,5-dihydro-3-methyl-5-oxo-1H-pyrazol-1-yl)-phenyl)-methansulfonamid
und aus 2-(4-Aminophenyl)-5-methyl-2,4-dihydro-pyrazol-3-on und Chlorameisensäuremethylester
N-(4-(4,5-dihydro-3-methyl-5-oxo-1H-pyrazol-1-yl)-phenyl)-carbaminsäuremethylester

### 12. N-(4-Phenyl-(4,5-dihydro-3-methyl-5-oxo-1H-pyrazol-1-yl)-acetamid

### (Reaktion der Aminogruppe des N-Substituenten des Pyrazols mit Essigsäurederivaten)

1.9 g 2-(4-Aminophenyl)-5-methyl-2,4-dihydro-pyrazol-3-on in 40 ml Tetrahydrofuran werden unter Eiskühlung und rühren mit 1.0 ml Acetanhydrid versetzt und 2 Stunden nachgerührt. Die Lösung wird im Vakuum eingeengt und der Rückstand wie üblich aufgearbeitet.
- Ausbeute:: 1.5 g N-(4-Phenyl-(4,5-dihydro-3-methyl-5-oxo-1H-pyrazol-1-yl)-acetamid (65 % der Theorie), Öl

Analog erhält man aus 2-(4-Aminophenyl)-5-methyl-2,4-dihydropyrazol-3-on und Trifluoracetanhydrid
N-(4-Phenyl-(4,5-dihydro-3-methyl-5-oxo-1H-pyrazol-1-yl)-trifluoracetamid
aus 2-(3-Aminophenyl)-5-methyl-2,4-dihydro-pyrazol-3-on
und Trifluoracetanhydrid
N-(3-Phenyl-(4,5-dihydro-3-methyl-5-oxo-1H-pyrazol-1-yl)-trifluoracetamid
aus 2-(3-Aminophenyl)-5-methyl-2,4-dihydro-pyrazol-3-on
und Acetanhydrid
N-(3-Phenyl-(4,5-dihydro-3-methyl-5-oxo-1H-pyrazol-1-yl)-acetamid aus 5-Methyl-2-(4-aminophenyl)-2,4-dihydro-pyrazol-3-on (analog Beispiel 10 hergestellt) und Acetanhydrid
N-(4-Phenyl(-4,5-dihydro-3-methyl-5-oxo-5H-pyrazol-1-yl))-acetamid) sowie durch Umsetzung mit 2-Ethylanilin
N-(4-(4-(2-Ethylanilinomethylen)-4,5-dihydro-3-methyl-5-oxo-1H-pyrazol-1-yl)-phenyl)-acetamid, FP: 230° C
13. aus 5-Methyl-2-(4-aminophenyl)-2,4-dihydro-pyrazol-3-on und Methansulfonylchlorid (Umsetzung erfolgt nach Herstellungsbeispiel 11, wobei das Methansulfonylchlorid in entsprechender molarer Menge eingesetzt wird)
   N-(4-(4,5-Dihydro-3-methyl-5-oxo-1H-pyrazol-1-yl)-phenyl)-N-methylsulfonylmethansulfonamid
   und 2-Ethylanilin
   N-(4-(4-(2-Ethylanilinomethylen)-4,5-dihydro-3-methyl-5-oxo-1H-pyrazol-1-yl)-phenyl)-N-methylsulfonylmethansulfonamid, FP: 268° C
14. aus 5-Methyl-2-(2-aminophenyl)-2,4-dihydro-pyrazol-3-on und Methansulfonylchlorid (Umsetzung erfolgt nach Herstellungsbeispiel 11)
   N-(4-(4,5-dihydro-3-methyl-5-oxo-1H-pyrazol-1-yl)-phenyl)-methansulfonamid
   und 2-Ethylanilin
   N-(2-(4-(2-Ethylanilinomethylen)-4,5-dihydro-3-methyl-5-oxo-1H-pyrazol-1-yl)-phenyl)-methansulfonamid, FP: 231° C
15. aus 5-Methyl-2-(3-aminophenyl)-2,4-dihydro-pyrazol-3-on und Trifluoracetanhydrid (Umsetzung erfolgt nach Herstellungsbeispiel 12)
   N-(3-Phenyl(-4,5-dihydro-3-methyl-5-oxo-1H-pyrazol-5-yl))-trifluoracetamid
   und 2-Ethylanilin
   N-(3-(4-(2-Ethylanilinomethylen)-4,5-dihydro-3-methyl-5-oxo-1H-pyrazol-5-yl)-phenyl)-trifluoracetamid, FP: 240° C
16. aus N,N-Dieethyl-4-hydrazinobenzolsulfonamid
   und Ethylacetoacetat
   N,N-Diethyl-4-(4,5-dihydro-3-methyl-5-oxo-1H-pyrazol-1-yl)-benzolsulfonamid
   und 2-Ethoxyanilin
   N,N-Diethyl-4-(4,5-dihydro-4-(2-ethoxyanilinomethylen)-3-methyl-5-oxo-1H-pyrazol-1-yl)-benzolsulfonamid, FP: 170° C
17. aus N,N-Dieethyl-4-hydrazinobenzolsulfonamid
   und Ethylacetoacetat
   N,N-Diethyl-4-(4,5-dihydro-3-methyl-5-oxo-1H-pyrazol-1-yl)-benzolsulfonamid
   und 2-Methoxyanilin
   N,N-Diethyl-4-(4,5-dihydro-4-(2-methoxyanilinomethylen)-3-methyl-5-oxo-1H-pyrazol-1-yl)-benzolsulfonamid, FP: 191 °C
18. aus N-Ethyl-4-hydrazinobenzolsulfonamid
   und Ethylacetoacetat
   N-Ethyl-4-(4,5-dihydro-3-methyl-5-oxo-1H-pyrazol-1-yl)-benzolsulfonamid
   und 2-Ethoxyanilin
   4-((2-Ethoxyanilinomethylen)-4,5-dihydro-3-methyl-5-oxo-1H-pyrazol-1-yl)-N-ethylbenzolsulfonamid, FP: 238° C
19. aus 5-Methyl-2-(4-aminophenyl)-2,4-dihydro-pyrazol-3-on und Chlorameisensäureethylester (nach Herstellungsbeispiel 11)
   N-(4-(4,5-dihydro-3-methyl-5-oxo-1H-pyrazol-1-yl)-phenylcarbaminsäureethylester
   und 2-Methoxyanilin
   N-(4-(4,5-Dihydro-4-(2-methoxyanilinomethylen)-3-methyl-5-oxo-1H-pyrazol-1-yl)-phenyl)-carbaminsaeureethylester, FP: 212° C
20. aus 5-Methyl-2-(4-aminophenyl)-2,4-dihydro-pyrazol-3-on und Propionylchlorid (nach Herstellungsbeispiel 11)
   N-(4-(4,5-dihydro-3-methyl-5-oxo-1H-pyrazol-1-yl)-phenyl)-propionamid
   und 2-Ethoxyanilin
   N-(4-(4-(2-Ethoxyanilinomethylen)-4,5-dihydro-3-methyl-5-oxo-1Hpyrazol-1-yl)-phenyl)-propionamid, FP: 208° C
21. aus 4-(1-Piperidyl)-sulfonylphenylhydrazin und Ethylacetoacetat
   5-Methyl-2-(4-(1-piperidylsulfonyl)-phenyl)-3H-pyrazol-3-on
   und 2-Ethoxyanilin
   4-(2-Ethoxyanilinomethylen)-2,4-dihydro-5-methyl-2-(4-(1-piperidylsulfonyl)-phenyl)-3H-pyrazol-3-on, FP: 252° C
22. aus N-tert.-Butyl-4-hydrazinobenzolsulfonamid und Ethylbutyrylacetat
   N-tert.-Butyl-4-(4,5-dihydro-3-propyl-5-oxo-1H-pyrazol-1-yl)-benzolsulfonamid
   und 2-Ethoxyanilin
   N-tert-Butyl-4-(4-(2-ethoxyanilinomethylen)-4,5-dihydro-3-propyl-5-oxo-1H-pyrazol-1-yl)-benzolsulfonamid, FP: 254° C

### 23. N-Acetyl-4-(4-(2-Ethoxyanilinomethylen)-4,5-dihydro-3-methyl-5 -oxo-1 H-pyrazol-1-yl)-benzolsulfonamid

### (Derivatisierung des N-Substituenten des Pyrazols nach der Ankondensation des Anilinderivats)

Zu einer Lösung von 1,0 g 4-(4-(2-Ethoxyanilinomethylen)-4,5-dihydro-3-methyl-5-oxo-1H-pyrazol-1-yl)benzolsulfonamid und 0,9 g Dimethylaminopyridin in 30 ml Pyridin tropft man unter Eiskühlung 0,17 ml Acetanhydrid und rührt 10 Stunden nach. Der nach dem Einengen im Vakuum erhaltenen Rückstand wird mit verdünnter Salzsäure versetzt, die ausgefallenen Kristalle abgesaugt und mit Ethanol verrieben.
- Ausbeute:: 0,47 g N-Acetyl-4-(4-(2-ethoxyanilinomethylen)-4,5-dihydro-3-methyl-5-oxo-1H-pyrazol-1-yl)-benzolsulfonamid (42,5 % der Theorie)
- Schmelzpunkt:: 282° C

24. aus 4-Hydrazinobenzolsulfonamid und Ethylacetoacetat
   4-(4,5-Dihydro-3-methyl-5-oxo-1H-pyrazol-1-yl)-benzolsulfonamid
   und 2-Ethoxyanilin
   4-(4-(2-Ethoxyanilinomethylen)-4,5-dihydro-3-methyl-5-oxo-1H-pyrazol-1-yl)-benzolsulfonamid, FP: 241° C
25. aus Phenylhydrazin und 5-Hydroxy-3-oxo-pentansäuremethylester
   5-(2-Hydroxyethyl)-2-phenyl-2,4-dihydropyrazol-3-on
   und 2-Ethylanilin
   4-(2-Ethylanilinomethylen)-2,4-dihydro-5-(2-hydroxyethyl)-2-phenyl-1H-pyrazol-3-on
26. aus 4-Methoxybenzylhydrazin und Ethylbutyrylacetat
   2,4-Dihydro-2-(4-methoxybenzyl)-5-propyl-3H-pyrazol-3-on
   und 2-Ethylanilin
   4-(2-Ethylanilinomethylen)-2,4-dihydro-2-(4-methoxybenzyl)-5-propyl-3H-pyrazol-3-on, Öl
27. aus 2-Propoxybenzylhydrazin und Ethylbutyrylacetat
   2,4-Dihydro-2-(2-Propoxybenzyl)-5-propyl-3H-pyrazol-3-on
   und 2-Ethylanilin
   4-(2-Ethylanilinomethylen)-2,4-dihydro-2-(2-propoxybenzyl)-5-propyl-3H-pyrazol-3-on, FP 75.2°C
28. aus 4-Bromphenylhydrazin und Ethylbutyrylacetat
   2-(4-Bromphenyl)2,4-dihydro-5-propyl-3H-pyrazol-3-on
   und 2-Ethylanilin
   2-(4-Bromphenyl)-4-(2-ethylanilinomethylen)-2,4-dihydro-5-propyl-3H-pyrazol-3-on, FP 126.9°C
29. aus 4-Nitrophenylhydrazin und Ethylbutyrylacetat
   2,4-Dihydro-2-(4-nitrophenyl)-5-propyl-3H-pyrazol-3-on
   und 2-Ethylanilin
   4-(2-Ethylanilinomethylen)-2,4-dihydro-2-(4-nitrophenyl)-5-propyl-3H-pyrazol-3-on, FP 211° C
30. aus 3-Hydrazinobenzolsulfonsäure und Ethylbutyrylacetat
   3-(4,5-Dihydro-5-oxo-3-propyl-1H-pyrazol-1-yl)-benzolsulfonsäure
   und 2-Ethylanilin
   3-(4-(2-Ethylanilinomethylen)-4,5-dihydro-5-oxo-3-propyl-1H-pyrazol-1-yl)-benzolsulfonsäure, FP 258.6° C
31. aus 4-Hydrazinobenzolsulfonsäure und Ethylbuturylacetat
   4-(4,5-Dihydro-5-oxo-3-propyl-1H-pyrazol-1-yl)-benzolsulfonsäure
   und 2-Ethylanilin
   4-(4-(2-Ethylanilinomethylen)-4,5-dihydro-5-oxo-3-propyl-1H-pyrazol-1-yl)-benzolsulfonsäure, FP 205.2° C
32. aus 4-Nitrophenylhydrazin und Diethyl-3-oxoglutarat
   2-(4,5-Dihydro-1-(4-nitrophenyl)-5-oxo-1H-pyrazol-3-yl)-essigsaeureethylester
   und 2-Ethylanilin
   2-(4-(2-Ethylanilinomethylen)-4,5-dihydro-1-(4-nitrophenyl)-5-oxo-1H-pyrazol-3-yl)-essigsaeureethylester, FP 224.5° C
33. aus 4-Hydrazinobenzoesäure und Ethylacetoacetat
   4-(4,5-Dihydro-5-oxo-3-methyl-1H-pyrazol-1-yl)-benzoesäure
   und 2-Ethylanilin
   4-(4-(2-Ethylanilinomethylen)-4,5-dihydro-5-oxo-3-methyl-1H-pyrazol-1-yl)-benzoesäure, FP 291° C
34. aus 2-Pyridylhydrazin und Ethylbutyrylacetat
   2,4-Dihydro-2-(2-pyridyl)-5-propyl-3H-pyrazol-3-on
   und 2-Ethylanilin
   4-(2-Ethylanilinomethylen)-2,4-dihydro-2-(2-pyridyl)-5-propyl-3H-pyrazol-3-on, FP 151°C
35. aus 2-Pyridylhydrazin und Ethylacetoacetat
   2,4-Dihydro-5-methyl-2-(2-pyridyl)-3H-pyrazol-3-on
   und 2-Ethylanilin
   4-(2-Ethylanilinomethylen)-2,4-dihydro-5-methyl-2-(2-pyridyl)-3H-pyrazol-3-on, FP 182.9° C
36. aus 4-Hydrazinobenzoesäure und Ethylbutyrylacetat
   4-(4,5-dihydro-5-oxo-3-propyl-1H-pyrazol-1-yl)-benzoesäure
   und 2-Ethylanilin
   4-(4-(2-Ethylanilinomethylen)-4,5-dihydro-5-oxo-3-propyl-1H-pyrazol-1-yl)-benzoesäure, FP 254.5 °C
37. aus 4-(4-(2-Ethylanilinomethylen)-4,5-dihydro-3-methyl-5-oxo-1H-pyrazol-1-yl)-benzoesäure
   und Hexylamin
   4-(4-(2-Ethylanilinomethylen)-4,5-dihydro-3-methyl-5-oxo-1H-pyrazol-1-yl)-N-hexylbenzamid, FP 62.1° C
38. aus 4-(4-(2-Ethylanilinomethylen)-4,5-dihydro-3-methyl-5-oxo-1H-pyrazol-1-yl)-benzoesäure und wäßriger Ammoniaklösung
   4-(4-(2-Ethylanilinomethylen)-4,5-dihydro-3-methyl-5-oxo-1H-pyrazol-1-yl)-benzamid, FP 225.2° C
39. aus 4-(4-(2-Ethylanilinomethylen)-4,5-dihydro-3-methyl-5-oxo-1H-pyrazol-1-yl)-benzoesäure und wäßriger N,N-Diethylaminlösung
   N,N-Diethyl-4-(4-(2-ethylanilinomethylen)-4,5-dihydro-3-methyl-5-oxo-1H-pyrazol-1-yl)-benzamid, FP 112° C
40. aus 4-Pyridylhydrazin und Ethylbutyrylacetat
   2,4-Dihydro-5-propyl-2-(4-pyridyl)-3H-pyrazol-3-on
   und 2-Ethylanilin
   4-(2-Ethylanilinomethylen)-2,4-dihydro-5-propyl-2-(4-pyridyl)-3H-pyrazol-3-on, FP 159.2° C
41. aus 4-Chlorphenylhydrazin und Ethylacetoacetat
   2-(4-Chlorphenyl)-2,4-dihydro-5-methyl-3H-pyrazol-3-on
   und 2-Ethylanilin
   2-(4-Chlorphenyl)-4-(2-ethylanilinomethylen)-2,4-dihydro-5-methyl-3H-pyrazol-3-on
42. aus 4-(4-(2-Ethylanilinomethylen)-4,5-dihydro-5-oxo-3-propyl-1H-pyrazol-1-yl)-benzoesäure und wäßriger Diethylaminlösung
   N,N-Diethyl-4-(4-(2-ethylanilinomethylen)-4,5-dihydro-5-oxo-3-propyl-1H-pyrazol-1-yl)-benzamid, FP 123 ° C
43. aus 4-(4-(2-Ethylanilinomethylen)-4,5-dihydro-5-oxo-3-propyl-1H-pyrazol-1-yl)-benzoesäure und Hexylamin
   4-(4-(2-Ethylanilinomethylen)-4,5-dihydro-5-oxo-3-propyl-1H-pyrazol-1-yl)-N-hexylbenzamid, FP 46.7° C
44. aus 4-(4-(2-Ethylanilinomethylen)-4,5-dihydro-5-oxo-3-propyl-1H-pyrazol-1-yl)-benzoesäure und wäßriger Ammoniaklösung
   4-(4-(2-Ethylanilinomethylen)-4,5-dihydro-5-oxo-3-propyl-1H-pyrazol-1-yl)-benzamid, FP 170° C
45. aus 4-Hydrazinobenzonitril und Ethylbutyrylacetat
   4-(4,5-Dihydro-5-oxo-3-propyl-1H-pyrazol-1-yl)-benzonitril
   und 2-Ethylanilin
   4-(4-(2-Ethylanilinomethylen)-4,5-dihydro-5-oxo-3-propyl-1H-pyrazol-1-yl)-benzonitril, FP 196.7° C
46. aus N,N-Diethyl-3-hydrazino-4-methoxybenzolsulfonamid und Ethylbutyrylacetat
   N,N-Diethyl-3-(4,5-dihydro-5-oxo-3-propyl-1H-pyrazol-1-yl)-4-methoxybenzolsulfonamid
   und 2-Ethylanilin
   N,N-Diethyl-3-(4-(2-ethylanilinomethylen)-4,5-dihydro-5-oxo-3-propyl-1H-pyrazol-1-yl)-4-methoxybenzolsulfonamid, Öl
47. aus 3-Hydrazinobenzonitril und Ethylacetoacetat
   3-(4,5-dihydro-3-methyl-5-oxo-1H-pyrazol-1-yl)-benzonitril
   und 2-Ethylanilin
   3-(4-(2-Ethylanilinomethylen)-4,5-dihydro-3-methyl-5-oxo-1H-pyrazol-1-yl)-benzonitril, FP 210.8 °C
48. aus N-Hexyl-3-hydrazino-4-propoxybenzolsulfonamid und Ethylacetoacetat
   3-(4,5-dihydro-3-methyl-5-oxo-1H-pyrazol-1-ylmethyl)-N-hexyl-4-propoxybenzolsulfonamid
   und 2-Ethylanilin
   3-(4-(2-Ethylanilinomethylen)-4,5-dihydro-3-methyl-5-oxo-1H-pyrazol-1-ylmethyl)-N-hexyl-4-propoxybenzolsulfonamid, Harz
49. aus 2-Hydrazinobenzoesäure und Ethylbutyrylacetat
   2-(4,5-Dihydro-5-oxo-3-propyl-1H-pyrazol-1-yl)-benzoesäure
   und 2-Ethylanilin
   2-(4-(2-Ethylanilinomethylen)-4,5-dihydro-5-oxo-3-propyl-1H-pyrazol-1-yl)-benzoesäure, FP 126.9° C
50. aus 4-(4-(2-Ethylanilinomethylen)-4,5-dihydro-5-oxo-3-propyl-1H-pyrazol-1-yl)-benzonitril
   und Trimethylzinnazid
   4-(2-Ethylanilinomethylen)-2,4-dihydro-5-propyl-2-(4-(1H-tetrazol-5-yl)-phenyl)-3H-pyrazol-3-on, FP 248.5° C
51. aus 3-Pyridylhydrazin und Ethylbutyrylacetat
   2,4-Dihydro-5-propyl-2-(3-pyridyl)-3H-pyrazol-3-on
   und 2-Ethylanilin
   4-(2-Ethylanilinomethylen)-2,4-dihydro-5-propyl-2-(3-pyridyl)-3H-pyrazol-3-on, FP 143.9° C
52. aus 3-(4-(2-Ethylanilinomethylen)-4,5-dihydro-3-methyl-5-oxo-1H-pyrazol-1-yl)-benzonitril und Trimethylzinnazid
   4-(2-Ethylanilinomethylen)-2,4-dihydro-5-methyl-2-(3-(1H-tetrazol-5-yl)-phenyl)-3H-pyrazol-3-on, FP 261.6°C
53. aus Hydrazinobenzoesäure und Ethylacetoacetat
   4-(4,5-Dihydro-3-methyl-5-oxo-1H-pyrazol-1-yl)-benzoesäure
   und 2-Trifluormethylanilin
   4-(4,5-Dihydro-3-methyl-5-oxo-4-(2-trifluormethylanilinomethylen)-1H-pyrazol-1-yl)-benzoesäure, FP 289.4° C
54. aus p-Hydrazinobenzoesäure und Diethyl-3-oxoglutarat
   4-(3-Ethoxycarbonylmethyl-4,5-dihydro-5-oxo-1H-pyrazol-1-yl)-benzoesaeure
   und 2-Ethylanilin
   4-(4-(2-Ethylanilinomethylen)-3-ethoxycarbonylmethyl-4,5-dihydro-5-oxo-1H-pyrazol-1-yl)-benzoesäure, FP 246° C
55. aus p-Hydrazinobenzoesäure und Ethylacetoacetat
   4-(4,5-Dihydro-3-methyl-5-oxo-1H-pyrazol-1-yl)-benzoesäure
   und 2-(2-Propinyloxy)anilin
   4-(4,5-Dihydro-3-methyl-5-oxo-4-(2-(2-propinyloxy)-anilinomethylen)-1H-pyrazol-1-yl)-benzoesäure, FP 267.9° C
56. aus p-Hydrazinobenzoesäure und Ethylacetoacetat
   4-(4,5-Dihydro-3-methyl-5-oxo-1H-pyrazol-1-yl)-benzoesäure
   und 2-Propoxyanilin
   4-(4,5-Dihydro-3-methyl-5-oxo-4-(2-propoxyanilinomethylen)-1H-pyrazol-1-yl)-benzoesäure, FP 259.6° C
57. aus p-Hydrazinobenzoesäure und Ethylacetoacetat
   4-(4,5-Dihydro-3-methyl-5-oxo-1H-pyrazol-1-yl)-benzoesäure
   und 2-(2-Propenyloxy)anilin
   4-(4,5-Dihydro-3-methyl-5-oxo-4-(2-(2-propenyloxy)-anilinomethylen)-1H-pyrazol-1-yl)-benzoesäure, FP 240.4° C
58. aus p-Hydrazinobenzoesäure und Ethylacetoacetat
   4-(4,5-Dihydro-3-methyl-5-oxo-1H-pyrazol-1-yl)-benzoesäure
   und 2-Methoxyanilin
   4-(4,5-Dihydro-4-(2-methoxyanilinomethylen)-3-methyl-5-oxo-1H-pyrazol-1-yl)-benzoesäure, FP >300° C
59. aus p-Hydrazinobenzoesäure und Ethylacetoacetat
   4-(4,5-Dihydro-3-methyl-5-oxo-1H-pyrazol-1-yl)-benzoesäure
   und 2-Isopropylanilin
   4-(4,5-Dihydro-4-(2-isopropylanilinomethylen)-3-methyl-5-oxo-1H-pyrazol-1-yl)-benzoesäure, FP 269.5° C
60. aus dem käuflichen 3-(4,5-Dihydro-3-methyl-5-oxo-1H-pyrazol-1-yl)-benzolsulfonamid und 2-Ethylanilin
   3-(4-(2-Ethylanilinomethylenaminomethylen)-4,5-dihydro-3-methyl-5-oxo-1H-pyrazol-1-yl)-benzolsulfonamid, FP 229.2° C
61. aus 2-(1-(4-Aminophenyl)-4,5-dihydro-5-oxo-1H-pyrazol-3-yl)essigsäureethylester und Trifluoracetanhydrid und nachfolgende Umsetzung mit Ethylanilin
   2-(4-(2-Ethylanilinomethylen)-4,5-dihydro-1-(4-trifluoracetamidophenyl)-5-oxo-1H-pyrazol-3-yl)-essigsäureethylester, FP: 197 °C.
62. aus 2-(1-(4-Aminophenyl)-4,5-dihydro-5-oxo-1H-pyrazol-3-yl)-essigsäureethylester und
   Chlorameisensäuremethylester und nachfolgende Umsetzung mit 2-Ethylanilin
   2-(1-(4-Methoxycarbonylaminophenyl)-4-((2-ethylanilino)-methylen)-4,5-dihydro-5-oxo-1H-pyrazol-3-yl)-essigsäureethylester, FP: 145 °C
63. aus 2-(4,5-Dihydro-1-(4-aminophenyl)-5-oxo-1H-pyrazol-3-yl)-essigsäureethylester und
   Methansulfonsäurechlorid und nachfolgender Umsetzung mit 2-Ethylanilin
   2-(4-((2-Ethylanilinomethylen)-4,5-dihydro-1-(4-methansulfonamidophenyl)-5-oxo-1H-pyrazol-3-yl)-essigsäureethylester, FP: 165 °C
64. Aus 2-(4,5-Dihydro-1-(4-aminophenyl)-5-oxo-1H-pyrazol-3-yl)-essigsäureethylester und
   Essigsäurechlorid und nachfolgender Umsetzung mit 2-Ethylanilin
   2-(1-(4-Acetamidophenyl)-4-((2-ethylanilino)-methylen)-4,5-dihydro-5-oxo-1H-pyrazol-3-yl)-essigsäureethylester, FP: 197 °C

In gleicher Weise wurden hergestellt:
2-(1-(4-(N,N-Diethylsulfamoyl)-phenyl)-4-(2-ethylanilinomethylen)-4,5-dihydro-5-oxo-1H-pyrazol-3-yl)-essigsäureethylester, FP: 146 ° C
2-(1-(4-(N,N-Diethylsulfamoyl)-phenyl)-4-(2-ethoxyanilinomethylen)-4,5-dihydro-5-oxo-1H-pyrazol-3-yl)-essigsäureethylester, FP: 127° C
2-(1-(4-Acetamidophenyl)-4-(2-ethylanilinomethylen)-4,5-dihydro-5-oxo-1Hpyrazol-3-yl)- essigsäureethylester, FP: 194° C
2-(4-(2-Ethylanilinomethylen)-4,5-dihydro-5-oxo-1-(4-trifluoroacetamidophenyl)-1H-pyrazol-3-yl)-essigsäureethylester, FP: 197° C
2-(4-(2-Ethylanilinomethylen)-4,5-dihydro-1-(4-methoxycarbonylaminophenyl)-5-oxo-1H-pyrazol-3-yl)- essigsäureethylester, FP: 144° C
2-(4-(2-Ethylanilinomethylen)-4,5-dihydro-1-(4-methansulfonamidophenyl)-5-oxo-1H-pyrazol-3-yl)- essigsäureethylester, FP: 165° C
2-(1-(4-Acetamidophenyl)-4-(2-ethylanilinomethylen)-4,5-dihydro-5-oxo-1H-pyrazol-3-yl)- essigsäureethylester, FP: 168° C
2-(4-(2-Ethylanilinomethylen)-4,5-dihydro-1-(4-methoxycarbonylaminophenyl)-5-oxo-1H-pyrazol-3-yl)-essigsäure, FP: 181° C
N-(3-(4-(2-Ethylanilinomethylen)-4,5-dihydro-5-oxo-3-propyl-1H-pyrazol-1-yl)-phenyl)-methansuifonamid, FP: 214° C
N-(3-(4-(2-Ethylanilinomethylen)-4,5-dihydro-5-oxo-3-propyl-1H-pyrazol-1-yl)-phenyl)-acetamid, FP: 181° C
N-(3-(4-(2-Ethylanilinomethylen)-4,5-dihydro-5-oxo-3-propyl-1H-pyrazol-1-yl)-phenyl)-carbaminsäuremethylester, FP: 203° C
2-(4-(2-Ethylanilinomethylen)-4,5-dihydro-5-oxo-1-(3-trifluoroacetamidophenyl)-1H-pyrazol-1-yl)- essigsäureethylester, FP: 190° C
2-(4-(2-Ethylanilinomethylen)-4,5-dihydro-1-(3-methansulfonamidophenyl)-5-oxo-1H-pyrazol-3-yl)-essigsäureethylester, FP: 174° C

Die nachfolgenden Beispiele betreffen pharmazeutische Zubereitungen:

### Beispiel A: Injektionsgläser

Eine Lösung von 100 g eines Wirkstoffs der Formel I und 5 g Dinatriumhydrogenphosphat werden in 3 l zweifach destilliertem Wasser mit 2 n Salzsäure auf pH 6.5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffs der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffs der Formel I, 9.38 g NaH₂PO₄·2H₂O, 28.48 g Na₂HPO₄·12H₂O und 0.1 g Benzalkoniumchlorid in 940 ml zweifach destilliertem Wasser. Man stellt auf pH 6.8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffs der Formel I mit 99.5 Vaseline unter aseptischen Bedingungen.

### Beispiel E: Tabletten

Ein Gemisch von 1 kg Wirkstoff der Formel I, 4 kg Laktose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zwiefach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der allgemeinen Formel (I) 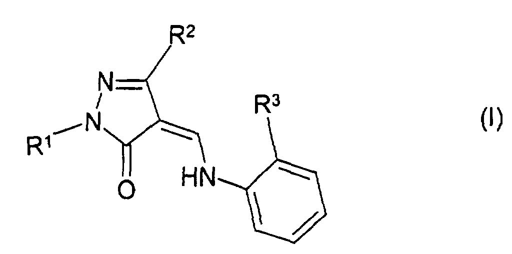 worin
R¹ Benzyl, Alkoxybenzyl mit 1 - 3 C-Atomen im Alkylteil, Phenyl, ein- bis dreifach durch Amino, Acyl, Halogen, Nitro, CN, AO-, Carboxyl, Sulfonyl, A-O-CO-, A-CO-NH-, A-CO-NA-, Carbamoyl, N-Alkylcarbamoyl, N,N-Dialkylcarbamoyl (mit 1 - 6 C-Atomen im Alkylteil), A-O-CO-NH-, A-O-CO-NA-, A-SO₂-, SO₂NR⁴R⁵ (R⁴ und R⁵ H oder Alkyl mit 1 - 6 C-Atomen oder NR⁴R⁵ ein 5- oder 6-gliedriger Ring, wahlweise mit anderen Heteroatomen wie N, S, O, der durch A substituiert sein kann), A-CO-NH-SO₂-, A-CO-NA-SO₂-, A-SO₂-NH-, A-SO₂-NA-, (A-SO₂-)₂ N-, Tetrazolyl oder Phosphonyl-substituiertes Phenyl; oder Pyridyl,
R² Alkyl mit 1 - 5 C-Atomen, Alkoxycarbonylalkyl, Hydroxyalkyl, Hydroxycarbonylalkyl,
R³ geradkettiges oder verzweigtes Alkyl mit 1 - 5 C-Atomen, geradkettiges oder verzweigtes Alkoxy mit 1 - 5 C-Atomen, durch Fluor oder Chlor substituiertes Alkyl,
A geradkettiges oder verzweigtes Alkyl mit 1 - 6 C-Atomen, geradkettiges oder verzweigtes, durch Fluor oder Chlor substituiertes Alkyl mit 1 - 6 C-Atomen
bedeuten oder deren Salze,
wobei 5-Methyl-2-phenyl-4-(o-tolylamino-methylen)-2,4-dihydropyrazol-3-on ausgeschlossen ist.

2. Verbindungen der allgemeinen Formel gemäß Anspruch 1,
worin
R¹ Benzyl, Alkoxybenzyl mit 1 - 3 C-Atomen im Alkylteil, Phenyl, ein- bis dreifach durch Amino, Halogen, Nitro, Cyano, Carboxyl, A-O-CO-, A-CO-NH-, A-CO-NA-, Carbamoyl, N-Alkylcarbamoyl, N,N-Dialkylcarbamoyl (mit 1 - 6 C-Atomen im Alkylteil), A-SO₂-NH-, A-SO₂-NA, Aminoacyl, HSO₃-, SO₂NR⁴R⁵ (R⁴ und R⁵ H oder Alkyl mit 1 - 6 C-Atomen oder NR⁴R⁵ ein 5- oder 6-gliedriger Ring, wahlweise mit anderen Heteroatomen wie N, S, O, der durch A sustituiert sein kann), A-CO-NH-SO₂-, A-CO-NH-SO₂- Tetrazolyl oder Phosphonyl substituiertes Phenyl; Pyridyl,
R² Hydroxyalkyl- und
R³ geradkettiges oder verzweigtes Alkyl- mit 1 - 5 C-Atomen, geradkettiges oder verzweigtes Alkoxy- mit 1 - 5 C-Atomen, durch Fluor oder Chlor substituiertes Alkyl,
A geradkettiges oder verzweigtes Alkyl mit 1 - 6 C-Atomen, geradkettiges oder verzweigtes, durch Fluor oder Chlor substituiertes Alkyl mit 1 - 6 C-Atomen
bedeuten oder deren Salze.

3. Eine Verbindung gemäß einem der Ansprüche 1 oder 2 ausgewählt aus der Gruppe:
N-3(4-(2-Ethylanilinomethylen)-4,5-dihydro-3-methyl-5-oxo-1H-pyrazol-1-yl)-phenyl)-carbaminsäuremethylester
4-((2-Ethoxyanilinomethylen)-4,5-dihydro-3-methyl-5-oxo-1H-pyrazol-1-yl)-N-ethylbenzolsulfonamid
2-(1-4-(N,N-Diethylsulfamoyl)-phenyl)-4-((2-ethylanilino)-methylen)-4,5-dihydro-5-oxo-1H-pyrazol-3-yl)-essigsäureethylester
2-(1-4-(N,N-Diethylsulfamoyl)-phenyl)-4-((2-ethoxylanilino)-methylen)-4,5-dihydro-5-oxo-1H-pyrazol-3-yl)-essigsäureethylester
2-(1-(4-Acetamidophenyl)-4-((2-ethylanilino)-methylen )-4,5-dihydro-5-oxo-1H-pyrazol-3-yl)-essigsäureethylester
2-(4-((2-Ethylanilino)-methylen)-4,5-dihydro-5-oxo-1-(4-trifluoracetamidophenyl)-1H-pyrazol-3-yl)-essigsäureethylester
2-(1-(4-Ethoxycarbonylaminophenyl)-4-((2-ethylanilino)-methylen)-4,5-dihydro-5-oxo-1H-pyrazol-3-yl)-essigsäureethylester
2-(4-((2-Ethylanilinomethylen)-4,5-dihydro-1-(4-methansulfonamidophenyl)-5-oxo-1H-pyrazol-3-yl)-essigsäureethylester
N-(3-(4-(2-Ethylanilinomethylen)-4,5-dihydro-3-methyl-5-oxo-5Hpyrazol-1-yl)-phenyl)-acetamid
N, N-Diethyl-4-(4-(2-ethylanilinomethylen)-4,5-dihydro-3-methyl-5-oxo-1H-pyrazol-1-yl)-benzolsulfonamid
N-Ethyl-4-(4-(2-ethylanilinomethylen)-4,5-dihydro-3-methyl-5-oxo-1Hpyrazol-1-yl)-benzolsulfonamid
4-(2-Ethoxyanilinomethylen)-2,4-dihydro-5-methyl-2-(4-(4-morpholinylsulfonyl)-phenyl)-3H-pyrazol-3-on
4-(2-Ethylanilinomethylen)-2,4-dihydro-5-methyl-2-(4-(4-methyl-1-piperazinylsulfonyl)-phenyl)-3H-pyrazol-3-on
N-(3-(4-(2-Ethylanilinomethylen)-4,5-dihydro-3-methyl-5-oxo-1H-pyrazol-1-yl)-phenyl)-methansulfonamid
N-(3-(4-(2-Ethylanilinomethylen)-4,5-dihydro-3-methyl-5-oxo-1Hpyrazol-1-yl)-phenyl)-trifluoracetamid
N-(4-(4-(2-Ethylanilinomethylen)-4,5-dihydro-3-methyl-5-oxo-1H-pyrazol-1-yl)-phenyl)-N-methylsulfonylmethansulfonamid
N,N-Diethyl-4-(4,5-dihydro-4-(2-ethoxyanilinomethylen)-3-methyl-5-oxo-1H-pyrazol-1-yl)-benzolsulfonamid
N,N-Diethyl-4-(4,5-dihydro-4-(2-methoxyanilinomethylen)-3-methyl-5-oxo-1H-pyrazol-1-yl)-benzolsulfonamid
3-(4-(2-Ethylanilinomethylen)-4,5-dihydro-5-oxo-3-propyl-1H-pyrazol-1-yl)-benzolsulfonsäure
4-(4-(2-Ethylanilinomethylen)-4,5-dihydro-5-oxo-3-propyl-1H-pyrazol-1-yl)-benzolsulfonsäure
2-(4-(2-Ethylanilinomethylen)-4,5-dihydro-1-(4-nitrophenyl)-5-oxo-1H-pyrazol-3-yl)-essigsaeureethylester
4-(4-(2-Ethylanilinomethylen)-4,5-dihydro-5-oxo-3-propyl-1H-pyrazol-1-yl)-benzoesäure
4-(4-(2-Ethylanilinomethylen)-4,5-dihydro-3-methyl-5-oxo-1H-pyrazol-1 -yl)-N-hexylbenzamid
4-(4-(2-Ethylanilinomethylen)-4,5-dihydro-3-methyl-5-oxo-1H-pyrazol-1-yl)-benzamid
N,N-Diethyl-4-(4-(2-ethylanilinomethylen)-4,5-dihydro-3-methyl-5-oxo-1H-pyrazol-1-yl)-benzamid
4-(2-Ethylanilinomethylen)-2,4-dihydro-5-propyl-2-(4-pyridyl)-3H-pyrazol-3-on
N,N-Diethyl-4-(4-(2-ethylanilinomethylen)-4,5-dihydro-5-oxo-3-propyl-1H-pyrazol-1-yl)-benzamid
4-(4-(2-Ethylanilinomethylen)-4,5-dihydro-5-oxo-3-propyl-1H-pyrazol-1-yl)-N-hexylbenzamid
4-(4-(2-Ethylanilinomethylen)-4,5-dihydro-5-oxo-3-propyl-1H-pyrazol-1-yl)-benzamid
4-(2-Ethylanilinomethylen)-2,4-dihydro-5-propyl-2-(4-(1H-tetrazol-5-yl)-phenyl)-3H-pyrazol-3-on
4-(2-Ethylanilinomethylen)-2,4-dihydro-5-methyl-2-(3-(1H-tetrazol-5-yl)-phenyl)-3H-pyrazol-3-on
4-(4,5-Dihydro-3-methyl-5-oxo-4-(2-trifluormethylanilinomethylen)-1H-pyrazol-1-yl)-benzoesäure
4-(4-(2-Ethylanilinomethylen)-3-ethoxycarbonylmethyl-4,5-dihydro-5-oxo-1H-pyrazol-1 -yl)-benzoesäure
4-(4,5-Dihydro-3-methyl-5-oxo-4-(2-propoxyanilinomethylen)-1Hpyrazol-1-yl)-benzoesäure
4-(4,5-Dihydro-4-(2-isopropylanilinomethylen)-3-methyl-5-oxo-1H-pyrazol-1-yl)-benzosäure
3-(4-(2-Ethylanilinomethylenaminomethylen)-4,5-dihydro-3-methyl-5-oxo-1H-pyrazol-1-yl)-benzolsulfonamid
2-(1-(4-Methoxycarbonylaminophenyl)-4-((2-ethylanilino)-methylen)-4,5-dihydro-5-oxo-1H-pyrazol-3-yl)-essigsäureethylester
2-(1-(4-(N,N-Diethylsulfamoyl)-phenyl)-4-(2-ethylanilinomethylen)-4,5-dihydro-5-oxo-1H-pyrazol-3-yl)-essigsäureethylester
2-(1-(4-(N,N-Diethylsulfamoyl)-phenyl)-4-(2-ethoxyanilinomethylen)-4,5-dihydro-5-oxo-1H-pyrazol-3-yl)-essigsäureethylester
2-(1-(4-Acetamidophenyl)-4-(2-ethylanilinomethylen)-4,5-dihydro-5-oxo-1H-pyrazol-3-yl)-essigsäureethylester
2-(4-(2-Ethylanilinomethylen)-4,5-dihydro-5-oxo-1-(4-trifluoroacetamidophenyl)1H-pyrazol-3-yl)-essigsäureethylester
2-(4-(2-Ethylanilinomethylen)-4,5-dihydro-1-(4-methoxycarbonylaminophenyl)-5-oxo-1H-pyrazol-3-yl)-essigsäureethylester
2-(4-(2-Ethylanilinomethylen)-4,5-dihydro-1-(4-methansulfonamidophenyl)-5-oxo-1H-pyrazol-3-yl)- essigsäureethylester
2-(1-(4-Acetamidophenyl)-4-(2-ethylanilinomethylen)-4,5-dihydro-5-oxo-1H-pyrazol-3-yl)-essigsäureethylester
2-(4-(2-Ethylanilinomethylen)-4,5-dihydro-1-(4-methoxycarbonylaminophenyl)-5-oxo-1H-pyrazol-3-yl)-essigsäure
N-(3-(4-(2-Ethylanilinomethylen)-4,5-dihydro-5-oxo-3-propyl-1H-pyrazol-1-yl)-phenyl)-methansulfonamid
N-(3-(4-(2-Ethylanilinomethylen)-4,5-dihydro-5-oxo-3-propyl-1H-pyrazol-1-yl)-phenyl)-acetamid
N-(3-(4-(2-Ethylanilinomethylen)-4,5-dihydro-5-oxo-3-propyl-1H-pyrazol-1-yl)-phenyl)-carbaminsäuremethylester
2-(4-(2-Ethylanilinomethylen)-4,5-dihydro-5-oxo-1-(3-trifluoracetamidophenyl)-1H-pyrazol-1-yl)-essigsäureethylester
2-(4-(2-Ethylanilinomethylen)-4,5-dihydro-1-(3-methansulfonamidophenyl)-5-oxo-1H-pyrazol-3-yl)-essigsäureethylester

4. Die Verbindung 4-(4,5-Dihydro-3-methyl-5-oxo-4-(2-(2-propinyloxy)-anilino-methylen)-1H-pyrazol-1-yl)-benzoesäure.

5. Arzneimittel der allgemeinen Formel I nach Anspruch 1, sowie deren physiologisch unbedenklichen Salze,
wobei 5-Methyl-2-phenyl-4-(o-tolylamino-methylen)-2,4-dihydropyrazol-3-on ausgeschlossen ist.

6. Verwendung der Arzneimittel gemäß Anspruch 5 zur Herstellung einer Arzneimittelformulierung zur selektiven Inhibierung der cGMP spezifischen Phosphodiesterase.

7. Verwendung der Verbindungen oder der physiologisch unbedenklichen Salze der Verbindungen gemäß Anspruch 5 zur Herstellung einer Arzneimittelformulierung.

8. Verwendung des Arzneimittels gemäß Anspruch 5 zur Herstellung einer Arzneimittelformulierung zur Behandlung von Krankheiten des Herz-Kreislaufsystems sowie der Herzinsuffizienz.

9. Pharmazeutische Zubereitung, **dadurch gekennzeichnet daß** sie mindestens eine Verbindung der Ansprüche 1 bis 4 und/oder mindestens eines ihrer physiologischen Salze oder ein Arzneimittel gemäß Anspruch 5 enthält.

10. Pharmazeutische Zubereitung, **dadurch gekennzeichnet, daß** sie mindestens ein Arzneimittel gemäß Anspruch 5 enthält und gemeinsam mit mindestens einem festen, flüssigen oder halbflüssigen Träger- oder Hilfsstoff in einer geeigneten Dosierungsform vorliegt.

11. Verfahren zur Herstellung der Verbindungen gemäß der Ansprüche 1 bis 4 oder des Arzneimittels gemäß Anspruch 5, **dadurch gekennzeichnet, daß** Verbindungen allgemeinen Formel II 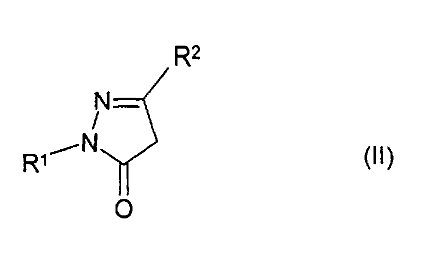 worin R¹ und R² die in den Ansprüchen 1 bis 2 oder 5 gegebenen Bedeutungen haben,
mit Formaldehyd-spendenden Gruppen aus der Gruppe Triazin, Dimethylformamid, Dimethylformamiddimethylacetal, Gold's Reagenz, Ameisensäurechlorid, Formamid oder Alkylderivate des Formamids mit 1-6 C-Atomen im Alkyl oder solcher der Gruppe der Trialkylorthoformiate, bevorzugt Trimethylorthoformiat, zu Verbindungen der allgemeinen Formel 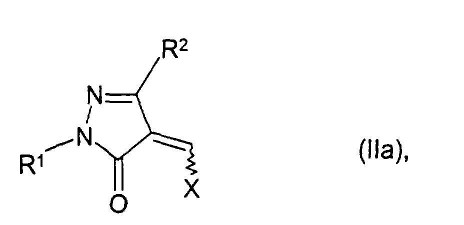 worin
X eine Amino- oder eine -O-Alkyl-Gruppe (mit 1-6 C-Atomen im Alkyl) bedeutet,
umgesetzt werden und diese, gegebenenfalls in situ, mit geeigneten Anilinderivaten der Formel 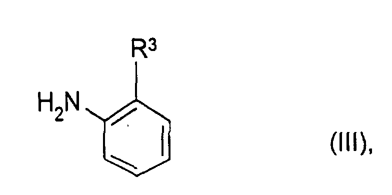 worin
R³ die in den Ansprüchen 1 bis 2 oder 5 gegebenen Bedeutungen besitzt,
oder deren Salze zu Verbindungen der Formel I umgesetzt werden und/oder daß in der Verbindung der Formel I ein oder mehrere Rest(e) in einen oder mehrere andere Rest(e) umwandelt werden
oder eine Verbindung der Formel II in Gegenwart einer Base aus der Gruppe Butyl- oder Methyllithium mit einem Aryl-Isocyanat umgesetzt wird.

## Claims

1. Compounds of the general formula (I) 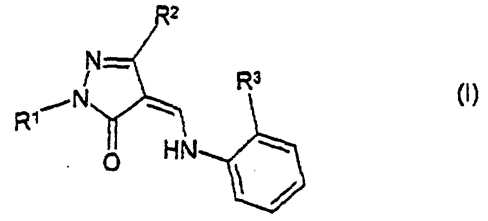 in which
R¹ is benzyl, alkoxybenzyl with 1-3 C atoms in the alkyl moiety, phenyl, phenyl which is substituted once to three times by amino, acyl, halogen, nitro, CN, AO-, carboxyl, sulphonyl, A-O-CO-, A-CO-NH-, A-CO-NA-, carbamoyl, N-alkylcarbamoyl, N,N-dialkylcarbamoyl (with 1-6 C atoms in the alkyl moiety), A-O-CO-NH-, A-O-CO-NA-, A-SO₂-, SO₂NR⁴R⁵ (R⁴ and R⁵ H or alkyl with 1-6 C atoms, or NR⁴R⁵ a 5- or 6-membered ring, optionally with other heteroatoms such as N, S, O, which may be substituted by A), A-CO-NH-SO₂-, A-CO-NA-SO₂-, A-SO₂-NH-, A-SO₂-NA-, (A-SO₂-)₂N-, tetrazolyl or phosphonyl; or pyridyl,
R² is alkyl with 1-5 C atoms, alkoxycarbonylalkyl, hydroxyalkyl, hydroxycarbonylalkyl,
R³ is straight-chain or branched alkyl with 1-5 C atoms, straight-chain or branched alkoxy with 1-5 C atoms, fluorine- or chlorine-substituted alkyl, A is straight-chain or branched alkyl with 1-6 C atoms, straight-chain or branched alkyl with 1-6 C atoms which is substituted by fluorine or chlorine,
or their salts, with exclusion of 5-methyl-2-phenyl-4-(o-tolylaminomethylene)-2,4-dihydropyrazol-3-one.

2. Compounds of the general formula according to Claim 1, in which
R¹ is benzyl, alkoxybenzyl with 1-3 C atoms in the alkyl moiety, phenyl, phenyl which is substituted once to three times by amino, halogen, nitro, cyano, carboxyl, A-O-CO-, A-CO-NH-, A-CO-NA-, carbamoyl, N-alkylcarbamoyl, N,N-dialkylcarbamoyl (with 1-6 C atoms in the alkyl moiety), A-SO₂-NH-, A-SO₂-NA-, aminoacyl, HSO₃-, SO₂NR⁴R⁵ (R⁴ and R⁵ H or alkyl with 1-6 C atoms, or NR⁴R⁵ a 5- or 6-membered ring, optionally with other heteroatoms such as N, S, O, which may be substituted by A), A-CO-NH-SO₂-, A-CO-NH-SO₂-, tetrazolyl or phosphonyl; or pyridyl,
R² is hydroxyalkyl and
R³ is straight-chain or branched alkyl with 1-5 C atoms, straight-chain or branched alkoxy with 1-5 C atoms, fluorine- or chlorine-substituted alkyl,
A is straight-chain or branched alkyl with 1-6 C atoms, straight-chain or branched alkyl with 1-6 C atoms which is substituted by fluorine or chlorine,
or their salts.

3. A compound according to either of Claims 1 and 2, selected from the group:
Methyl N-(3-(4-(2-ethylanilinomethylene)-4,5-dihydro-3-methyl-5-oxo-1H-pyrazol-1-yl)phenyl)carbamate
4-((2-Ethoxyanilinomethylene)-4,5-dihydro-3-methyl-5-oxo-1H-pyrazol-1-yl)-N-ethylbenzenesulphonamide
Ethyl 2-(1-(4-(N,N-diethylsulphamoyl)phenyl)-4-(2-ethylanilinomethylene)-4,5-dihydro-5-oxo-1H-pyrazol-3-yl)acetate
Ethyl 2-(1-(4-(N,N-diethylsulphamoyl)phenyl)-4-(2-ethoxyanilinomethylene)-4,5-dihydro-5-oxo-1H-pyrazol-3-yl)acetate
Ethyl 2-(1-(4-acetamidophenyl)-4-(2-ethylanilino-methylene)-4,5-dihydro-5-oxo-1H-pyrazol-3-yl)acetate
Ethyl 2-(4-(2-ethylanilinomethylene)-4,5-dihydro-5-oxo-1-(4-trifluoroacetamidophenyl)-1H-pyrazol-3-yl)acetate
Ethyl 2-(1-(4-ethoxycarbonylaminophenyl)-4-(2-ethylanilinomethylene)-4,5-dihydro-5-oxo-1H-pyrazol-3-yl)-acetate
Ethyl 2-(4-(2-ethylanilinomethylene)-4,5-dihydro-1-(4-methanesulphonamidophenyl)-5-oxo-1H-pyrazol-3-yl)acetate
N-(3-(4-(2-Ethylanilinomethylene)-4,5-dihydro-3-methyl-5-oxo-5H-pyrazol-1-yl)phenyl)acetamide
N,N-Diethyl-4-(4-(2-ethylanilinomethylene)-4,5-dihydro-3-methyl-5-oxo-1H-pyrazol-1-yl)benzenesulphonamide
N-Ethyl-4-(4-(2-ethylanilinomethylene)-4,5-dihydro-3-methyl-5-oxo-1H-pyrazol-1-yl)benzenesulphonamide
4-(2-Ethoxyanilinomethylene)-2,4-dihydro-5-methyl-2-(4-(4-morpholinylsulphonyl)phenyl)-3H-pyrazol-3-one
4-(2-Ethylanilinomethylene)-2,4-dihydro-5-methyl-2-(4-(4-methyl-1-piperazinylsulphonyl)phenyl)-3H-pyrazol-3-one
N-(3-(4-(2-Ethylanilinomethylene)-4,5-dihydro-3-methyl-5-oxo-1H-pyrazol-1-yl)phenyl)methanesulphonamide
N-(3-(4-(2-Ethylanilinomethylene)-4,5-dihydro-3-methyl-5-oxo-1H-pyrazol-1-yl)phenyl)trifluoroacetamide .
N-(4-(4-(2-Ethylanilinomethylene)-4,5-dihydro-3-methyl-5-oxo-1H-pyrazol-1-yl)phenyl)-N-methylsulphonylmethanesulphonamide
N,N-Diethyl-4-(4,5-dihydro-4-(2-ethoxyanilinomethylene)-3-methyl-5-oxo-1H-pyrazol-1-yl)benzenesulphonamide
N,N-Diethyl-4- (4,5-dihydro-4-(2-methoxyanilinomethylene)-3-methyl-5-oxo-1H-pyrazol-1-yl)benzenesulphonamide
3-(4-(2-Ethylanilinomethylene)-4,5-dihydro-5-oxo-3-propyl-1H-pyrazol-1-yl)benzenesulphonic acid
4-(4-(2-Ethylanilinomethylene)-4,5-dihydro-5-oxo-3-propyl-1H-pyrazol-1-yl)benzenesulphonic acid
Ethyl 2-(4-(2-ethylanilinomethylene)-4,5-dihydro-1-(4-nitrophenyl)-5-oxo-1H-pyrazol-3-yl)acetate
4-(4-(2-Ethylanilinomethylene)-4,5-dihydro-5-oxo-3-propyl-1H-pyrazol-1-yl)benzoic acid
4-(4-(2-Ethylanilinomethylene)-4,5-dihydro-3-methyl-5-oxo-1H-pyrazol-1-yl)-N-hexylbenzamide
4(4-(2-Ethylanilinomethylene)-4,5-dihydro-3-methyl-5-oxo-1H-pyrazol-1-yl)benzamide
N,N-Diethyl-4-(4-(2-ethylanilinomethylene)-4,5-dihydro-3-methyl-5-oxo-1H-pyrazol-1-yl)benzamide
4-(2-Ethylanilinomethylene)-2,4-dihydro-5-propyl-2-(4-pyridyl)-3H-pyrazol-3-one
N,N-Diethyl-4-(4-(2-ethylanilinomethylene)-4,5-dihydro-5-oxo-3-propyl-1H-pyrazol-1-yl)benzamide
4-(4-(2-Ethylanilinomethylene)-4,5-dihydro-5-oxo-3-propyl-1H-pyrazol-1-yl)-N-hexylbenzamide
4-(4-(2-Ethylanilinomethylene)-4,5-dihydro-5-oxo-3-propyl-1H-pyrazol-1-yl)benzamide
4-(2-Ethylanilinomethylene)-2,4-dihydro-5-propyl-2-(4-(1H-tetrazol-5-yl)phenyl)-3H-pyrazol-3-one
4-(2-Ethylanilinomethylene)-2,4-dihydro-5-methyl-2-(3-(1H-tetrazol-5-yl)phenyl) -3H-pyrazol-3-one
4- (4-(2-Ethylanilinomethylene)-3-ethoxycarbonylmethyl-4,5-dihydro-5-oxo-1H-pyrazol-1-yl)benzoic acid
4-(4,5-Dihydro-3-methyl-5-oxo-4-(2-(2-propynyloxy)-anilinomethylene)-1H-pyrazol-1-yl)benzoic acid
4-(4,5-Dihydro-3-methyl-5-oxo-4- (2-propoxyanilinomethylene)-1H-pyrazol-1-yl)benzoic acid
4-(4,5-Dihydro-4-(2-isopropylanilinomethylene)-3-methyl-5-oxo-1H-pyrazol-1-yl)benzoic acid
3-(4-(2-Ethylanilinomethyleneaminomethylene)-4,5-dihydro-3-methyl-5-oxo-1H-pyrazol-1-yl)benzenesulphonamide
Ethyl 2-(1-(4-methoxycarbonylaminophenyl)-4-((2-ethylanilino)-methylene)-4,5-dihydro-5-oxo-1H-pyrazol-3-yl)acetate
Ethyl 2-(1-(4-(N,N-diethylsulphamoyl)-phenyl-4-(2-ethylanilinomethylene)4,5-dihydro-5-oxo-1H-pyrazol-3-yl)-acetate
Ethyl 2-(1-(4-(N,N-diethylsulphamoyl)-phenyl)-4-(2-ethoxyanilinomethylene)-4,5-dihydro-5-oxo-1H-pyrazol-3-yl)acetate
Ethyl 2-(1-(4-acetamidophenyl)-4-(2-ethylanilinomethylene)-4,5-dihydro-5-oxo-1H-pyrazol-3-yl)acetate
Ethyl 2-(4-(2-ethylanilinomethylene)-4,5-dihydro-5-oxo-1-(4-trifluoroacetamidophenyl)1H-pyrazol-3-yl)acetate
Ethyl 2-(4-(2-ethylanilinomethylene)-4,5-dihydro-1-(4-methoxycarbonylaminophenyl)-5-oxo-1H-pyrazol-3-yl)acetate
Ethyl 2-(4-(2-ethylanilinomethylene)-4,5-dihydro-l-(4-methanesulphonamidophenyl)-5-oxo-1H-pyrazol-3-yl)acetate
Ethyl 2-(1-(4-acetamidophenyl)-4-(2-ethylanilinomethylene)-4,5-dihydro-5-oxo-1H-pyrazol-3-yl)acetate
2-(4-(2-Ethylanilinomethylene)-4,5-dihydro-1-(4-methoxycarbonylaminophenyl)-5-oxo-1H-pyrazol-3-yl)acetic acid
N-(3-(4-(2-Ethylanilinomethylene)-4,5-dihydro-5-oxo-3-propyl-1H-pyrazol-1-yl)-phenyl)-methanesulphonamide
N-(3-(4-(2-Ethylanilinomethylene)-4,5-dihydro-5-oxo-3-propyl-1H-pyrazol-1-yl)-phenyl)-acetamide
Methyl N-(3-(4-(2-ethylanilinomethylene)-4,5-dihydro-5-oxo-3-propyl-1H-pyrazol-1-yl)-phenyl-carbamate
Ethyl 2-(4-(2-Ethylanilinomethylene)-4,5-dihydro-5-oxo-1-(3-trifluoracetamidophenyl)-1H-pyrazol-1-yl)acetate
Ethyl 2-(4-(2-ethylanilinomethylene)-4,5-dihydro-1-(3-methanesulphonamidophenyl)-5-oxo-1H-pyrazol-3-yl)acetate

4. The compound 4-(4,5-dihydro-3-methyl-5-oxo-4-(2-(2-propynyloxy)-anilinomethylene)-1H-pyrazol-1-yl)-benzoic acid.

5. Medicaments of the general formula I according to Claim 1, and their physiologically acceptable salts, with exclusion of 5-methyl-2-phenyl-4-(o-tolylaminomethylene)-2,4-dihydropyrazol-3-one

6. Use of the medicaments according to Claim 5 for the production of a medicament formulation for selective inhibition of cGMP-specific phosphodiesterase.

7. Use of the compounds or of the physiologically acceptable salts of the compounds according to Claim 5 for the production of a medicament formulation.

8. Use of the medicament according to Claim 5 for the production of a medicament formulation for the treatment of disorders of the cardiovascular system and of heart failure.

9. Pharmaceutical preparation, **characterized in that** it contains at least one compound of Claims 1 to 4 and/or at least one of its physiological salts or a medicament according to Claim 5.

10. Pharmaceutical preparation, **characterized in that** it contains at least one medicament according to Claim 5 and is in a suitable dosage form together with at least one solid, liquid or semiliquid vehicle or auxiliary.

11. Process for the preparation of the compounds according to Claims 1 to 4 or of the medicament according to Claim 5, **characterized in that** compounds of the general formula II 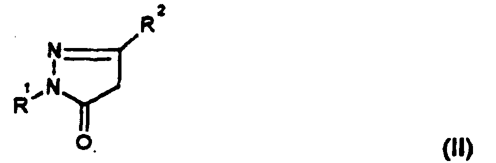 in which R¹ and R² have the meanings given in Claims 1 to 2 or 5,
are reacted with formaldehyde-donating compounds from the group of triazine, dimethylformamide, dimethylformamide dimethyl acetal, Gold's reagent, formyl chloride, formamide or alkyl derivatives of formamide with 1-6 C atoms in the alkyl or those of the group of trialkyl orthoformates, preferably trimethyl orthoformate, to give compounds of the general formula 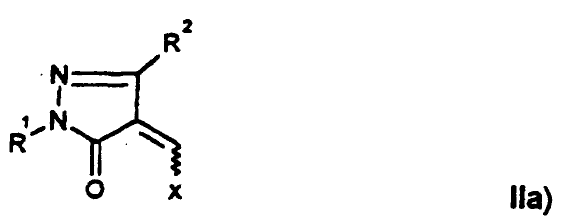 in which
X is an amino or an O-alkyl group (with 1-6 C atoms in the alkyl),
and the latter are reacted, where appropriate in situ, with suitable aniline derivatives of the formula 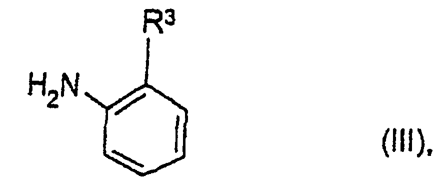 in which
R³ has the meanings given in Claims 1 to 2 or 5,
or their salts to give compounds of the formula I 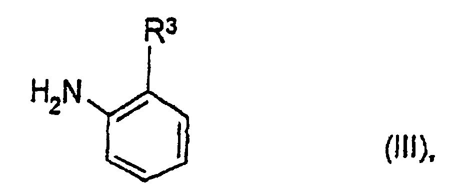 and/or **in that** one or more radical(s) in the compound of the formula I are converted into one or more other radical(s), or a compound of the formula II is reacted with an aryl isocyanate in the presence of a base from the group of butyl- or methyllithium.

## Revendications

1. Composés de formule générale (I) 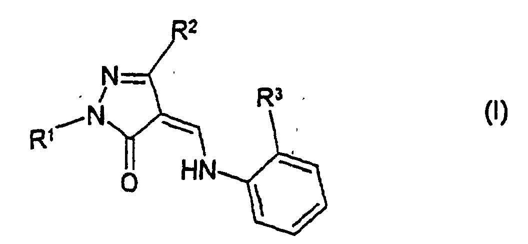 dans laquelle
R¹ représente un groupe benzyle, alcoxybenzyle ayant de 1 à 3 atomes de carbone dans le fragment alkyle, le groupe phényle, un groupe phényle une à trois fois substitué par un ou des atomes d'halogène ou groupes amino, acyle, nitro, CN, AO-, carboxy, sulfonyle, A-O-CO-, A-CO-NH-, A-CO-NA-, carbamoyle, N-alkylcarbamoyle, N,N-dialkylcarbamoyle (ayant de 1 à 6 atomes de carbone dans le fragment alkyle), A-O-CO-NH-, A-O-CO-NA-, A-SO₂-, SO₂NR⁴R⁵ (R⁴ et R⁵ représentant H ou un groupe alkyle ayant de 1 à 6 atomes de carbone, ou NR⁴R⁵ formant un cycle à 5 ou 6 chaînons, comportant éventuellement d'autres hétéroatomes tels que N, S, O, qui peut être substitué par A), A-CO-NH-SO₂-, A-CO-NA-SO₂-, A-SO₂-NH-, A-SO₂-NA-, (A-SO₂)₂N-, tétrazolyle ou phosphonyle ; ou pyridyle,
R² représente un groupe alkyle ayant de 1 à 5 atomes de carbone, alcoxycarbonylalkyle, hydroxyalkyle, hydroxycarbonylalkyle,
R³ représente un groupe alkyle à chaîne droite ou ramifié ayant de 1 à 5 atomes de carbone,
un groupe alcoxy à chaîne droite ou ramifié ayant de 1 à 5 atomes de carbone,
un groupe alkyle substitué par le fluor ou le chlore,
A représente un groupe alkyle à chaîne droite ou ramifié ayant de 1 à 6 atomes de carbone,
un groupe alkyle à chaîne droite ou ramifié ayant de 1 à 6 atomes de carbone, substitué par le fluor ou le chlore,
ou sels de tels composés,
à l'exclusion de la 5-méthyl-2-phényl-4-(o-tolylaminométhylène)-2,4-dihydropyrazol-3-one.

2. Composés de formule générale selon la revendication 1, dans lesquels
R¹ représente un groupe benzyle, alcoxybenzyle ayant de 1 à 3 atomes de carbone dans le fragment alkyle, le groupe phényle, un groupe phényle une à trois fois substitué par un ou des atomes d'halogène ou groupes amino, nitro, cyano, carboxy, A-O-CO-, A-CO-NH-, A-CO-NA-, carbamoyle, N-alkylcarbamoyle, N,N-dialkylcarbamoyle (ayant de 1 à 6 atomes de carbone dans le fragment alkyle), A-SO₂-NH-, A-SO₂-NA, aminoacyle, HSO₃-, SO₂NR⁴R⁵ (R⁴ et R⁵ représentant H ou un groupe alkyle ayant de 1 à 6 atomes de carbone, ou NR⁴R⁵ formant un cycle à 5 ou 6 chaînons, comportant éventuellement d'autres hétéroatomes tels que N, S, O, qui peut être substitué par A), A-CO-NH-SO₂-, A-CO-NA-SO₂-, tétrazolyle ou phosphonyle ; pyridyle,
R² représente un groupe hydroxyalkyle et
R³ représente un groupe alkyle à chaîne droite ou ramifié ayant de 1 à 5 atomes de carbone,
un groupe alcoxy à chaîne droite ou ramifié ayant de 1 à 5 atomes de carbone,
un groupe alkyle substitué par le fluor ou le chlore,
A représente un groupe alkyle à chaîne droite ou ramifié ayant de 1 à 6 atomes de carbone,
un groupe alkyle à chaîne droite ou ramifié ayant de 1 à 6 atomes de carbone, substitué par le fluor ou le chlore,
ou sels de tels composés.

3. Composé selon la revendication 1 ou 2, choisi dans le groupe constitué par les composés suivants :
N-3-(4-(2-éthylanilinométhylène)-4,5-dihydro-3-méthyl-5-oxo-1H-pyrazol-1-yl)-phényl)carbamate de méthyle
4-((2-éthoxyanilinométhylène)-4,5-dihydro-3-méthyl-5-oxo-1H-pyrazol-1-yl)-N-éthylbenzènesulfonamide
2-(1-(4-(N,N-diéthylsulfamoyl)phényl)-4-((2-éthylanilino)méthylène)-4,5-dihydro-5-oxo-1H-pyrazol-3-yl)acétate d'éthyle
2-(1-(4-(N,N-diéthylsulfamoyl)phényl)-4-((2-éthoxyanliino)méthylène)-4,5-dihydro-5-oxo-1H-pyrazol-3-yl)acétate d'éthyle
2-(1-(4-acétamidophényl)-4-((2-éthylanilino)méthylène)-4,5-dihydro-5-oxo-1H-pyrazol-3-yl)acétate d'éthyle
2-(4-((2-éthylanilino)méthylène)-4,5-dihydro-5-oxo-1-(4-trifluoroacétamidophényl)-1H-pyrazol-3-yl)acétate d'éthyle
2-(1-(4-éthoxycarbonylaminophényl)-4-((2-éthylanilino)méthylène)-4,5-dihydro-5-oxo-1H-pyrazol-3-yl)acétate d'éthyle
2-(4-((2-éthylanilinométhylène)-4,5-dihydro-1-(4-méthanesulfonamidophényl)-5-oxo-1H-pyrazol-3-yl)acétate d'éthyle
N-(3-(4-(2-éthylanilinométhylène)-4,5-dihydro-3-méthyl-5-oxo-5H-pyrazol-1-yl)-phénylacétamide
N,N-diéthyl-4-(4-(2-éthylanilinométhylène)-4,5-dihydro-3-méthyl-5-oxo-1H-pyrazol-1-yl)benzènesulfonamide
N-éthyl-4-(4-(2-éthylanilinométhylène)-4,5-dihydro-3-méthyl-5-oxo-1H-pyrazol-1-yl)benzènesulfonamide
4-(2-éthoxyanilinométhylène)-2,4-dihydro-5-méthyl-2-(4-(4-morpholinylsulfonyl)-phényl)-3H-pyrazol-3-one
4-(2-éthylanilinométhylène)-2,4-dihydro-5-méthyl-2-(4-(4-méthyl-1-pipérazinylsulfonyl)phényl)-3H-pyrazol-3-one
N-(3-(4-(2-éthylanilinométhylène)-4,5-dihydro-3-méthyl-5-oxo-1H-pyrazol-1-yl)-phényl)méthanesulfonamide
N-(3-(4-(2-éthylanilinométhylène)-4,5-dihydro-3-méthyl-5-oxo-1H-pyrazol-1-yl)-phényl)trifluoroacétamide
N-(4-(4-(2-éthylanilinométhylène)-4,5-dihydro-3-méthyl-5-oxo-1H-pyrazol-1-yl)-phényl)-N-méthylsulfonylméthanesulfonamide
N,N-diéthyl-4-(4,5-dihydro-4-(2-éthoxyanilinométhylène)-3-méthyl-5-oxo-1H-pyrazol-1-yl)benzènesulfonamide
N,N-diéthyl-4-(4,5-dihydro-4-(2-méthoxyanilinométhylène)-3-méthyl-5-oxo-1H-pyrazol-1-yl)benzènesulfonamide
acide 3-(4-(2-éthylanilinométhylène)-4,5-dihydro-5-oxo-3-propyl-1H-pyrazol-1-yl)benzènesulfonique
acide 4-(4-(2-éthylanilinométhylène)-4,5-dihydro-5-oxo-3-propyl-1H-pyrazol-1-yl)benzènesulfonique
2-(4-(2-éthylanilinométhylène)-4,5-dihydro-1-(4-nitrophényl)-5-oxo-1H-pyrazol-3-yl)acétate d'éthyle
acide 4-(4-(2-éthylanilinométhylène)-4,5-dihydro-5-oxo-3-propyl-1H-pyrazol-1-yl)benzoïque
4-(4-(2-éthylanilinométhylène)-4,5-dihydro-3-méthyl-5-oxo-1H-pyrazol-1-yl)-N-hexylbenzamide
4-(4-(2-éthylanilinométhylène)-4,5-dihydro-3-méthyl-5-oxo-1H-pyrazol-1-yl)-benzamide
N,N-diéthyl-4-(4-(2-éthylanilinométhylène)-4,5-dihydro-3-méthyl-5-oxo-1H-pyrazol-1-yl)benzamide
4-(2-éthylanilinométhylène)-2,4-dihydro-5-propyl-2-(4-pyridyl)-3H-pyrazol-3-one
N,N-diéthyl-4-(4-(2-éthylanilinométhylène)-4,5-dihydro-5-oxo-3-propyl-1H-pyrazol-1-yl)benzamide
4-(4-(2-éthylanilinométhylène)-4,5-dihydro-5-oxo-3-propyl-1H-pyrazol-1-yl)-N-hexylbenzamide
4-(4-(2-éthylanilinométhylène)-4,5-dihydro-5-oxo-3-propyl-1H-pyrazol-1-yl)-benzamide
4-(2-éthylanilinométhylène)-2,4-dihydro-5-propyl-2-(4-(1H-tétrazol-5-yl)phényl)-3H-pyrazol-3-one
4-(2-éthylanilinométhylène)-2,4-dihydro-5-méthyl-2-(3-(1H-tétrazol-5-yl)phényl)-3H-pyrazol-3-one
acide 4-(4,5-dihydro-3-méthyl-5-oxo-4-(2-trifluorométhylanilinométhylène)-1H-pyrazol-1-yl)benzoïque
acide 4-(4-(2-éthylanilinométhylène)-3-éthoxycarbonylméthyl-4,5-dihydro-5-oxo-1H-pyrazol-1-yl)benzoïque
acide 4-(4,5-dihydro-3-méthyl-5-oxo-4-(2-propoxyanilinométhylène)-1H-pyrazol-1-yl)benzoïque
acide 4-(4,5-dihydro-4-(2-isopropylanilinométhylène)-3-méthyl-5-oxo-1H-pyrazol-1-yl)benzoïque
3-(4-(2-éthylanilinométhylène-aminométhylène)-4,5-dihydro-3-méthyl-5-oxo-1H-pyrazol-1-yl)benzènesulfonamide
2-(1-(4-méthoxycarbonylaminophényl)-4-((2-éthylanilino)méthylène)-4,5-dihydro-5-oxo-1H-pyrazol-3-yl)acétate d'éthyle
2-(1-(4-(N,N-diéthylsulfamoyl)phényl)-4-(2-éthylanilinométhylène)-4,5-dihydro-5-oxo-1H-pyrazol-3-yl)acétate d'éthyle
2-(1-(4-(N,N-diéthylsulfamoyl)phényl)-4-(2-éthoxyanilinométhylène)-4,5-dihydro-5-oxo-1H-pyrazol-3-yl)acétate d'éthyle
2-(1-(4-acétamidophényl)-4-(2-éthylanilinométhylène)-4,5-dihydro-5-oxo-1H-pyrazol-3-yl)acétate d'éthyle
2-(4-(2-éthylanilinométhylène)-4,5-dihydro-5-oxo-1-(4-trifluoroacétamidophényl)-1H-pyrazol-3-yl)acétate d'éthyle
2-(4-(2-éthylanilinométhylène)-4,5-dihydro-1-(4-méthoxycarbonylaminophényl)-5-oxo-1H-pyrazol-3-yl)acétate d'éthyle
2-(4-(2-éthylanilinométhylène)-4,5-dihydro-1-(4-méthanesulfonamidophényl)-5-oxo-1H-pyrazol-3-yl)acétate d'éthyle
2-(1-(4-acétamidophényl)-4-(2-éthylanilinométhylène)-4,5-dihydro-5-oxo-1H-pyrazol-3-yl)acétate d'éthyle
acide 2-(4-(2-éthylanilinométhylène)-4,5-dihydro-1-(4-méthoxycarbonylaminophényl)-5-oxo-1H-pyrazol-3-yl)acétique
N-(3-(4-(2-éthylanilinométhylène)-4,5-dihydro-5-oxo-3-propyl-1H-pyrazol-1-yl)phényl)méthanesulfonamide
N-(3-(4-(2-éthylanilinométhylène)-4,5-dihydro-5-oxo-3-propyl-1H-pyrazol-1-yl)phényl)acétamide
N-(3-(4-(2-éthylanilinométhylène)-4,5-dihydro-5-oxo-3-propyl-1H-pyrazol-1-yl)phényl)carbamate de méthyle
2-(4-(2-éthylanilinométhylène)-4,5-dihydro-5-oxo-1-(3-trifluoroacétamidophényl)-1H-pyrazol-1-yl)acétate d'éthyle
2-(4-(2-éthylanilinométhylène)-4,5-dihydro-1-(3-méthanesulfonamidophényl)-5-oxo-1H-pyrazol-3-yl)acétate d'éthyle.

4. Composé qui est l'acide 4-(4,5-dihydro-3-méthyl-5-oxo-4-(2-(2-propynyloxy)anilinométhylène)-1H-pyrazol-1-yl)benzoïque.

5. Médicaments de formule générale I selon la revendication 1 ainsi que leurs sels physiologiquement acceptables, à l'exclusion de la 5-méthyl-2-phényl-4-(o-tolyiaminomèthylène)-2,4-dihydropyrazol-3-one.

6. Utilisation des médicaments selon la revendication 5, pour la fabrication d'une composition pharmaceutique destinée à l'inhibition sélective des phosphodiestérases spécifiques de GMPc.

7. Utilisation des composés ou des sels physiologiquement acceptables des composés selon la revendication 5, pour la fabrication d'une composition pharmaceutique.

8. Utilisation du médicament selon la revendication 5 pour la fabrication d'une composition pharmaceutique destinée au traitement de maladies du système cardiovasculaire ainsi que de l'insuffisance cardiaque.

9. Préparation pharmaceutique, **caractérisée en ce qu'**elle contient au moins un composé des revendications 1 à 4, et/ou au moins un de ses sels physiologiques ou un médicament selon la revendication 5.

10. Préparation pharmaceutique, **caractérisée en ce qu'**elle contient au moins un médicament selon la revendication 5 et, conjointement avec au moins un véhicule ou adjuvant solide, liquide ou semi-liquide, se trouve sous une forme d'administration appropriée.

11. Procédé pour la préparation des composés selon les revendications 1 à 4 ou du médicament selon la revendication 5, **caractérisé en ce qu'**on fait réagir des composés de formule générale II 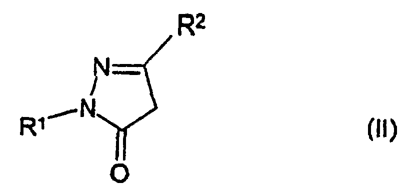 dans laquelle R¹ et R² ont les significations données dans les revendications 1 et 2 ou 5
avec des groupes donneurs de formaldéhyde, choisis dans l'ensemble constitué par la triazine, le diméthylformamide, le diméthylformamide-diméthylacétal, le réactif de Gold, le chlorure de formyle, le formamide ou des dérivés alkyliques du formamide ayant de 1 à 6 atomes de carbone dans le fragment alkyle, ou ceux du groupe des orthoformiates de trialkyle, de préférence l'orthoformiate de triméthyle, pour aboutir à des composés de formule générale 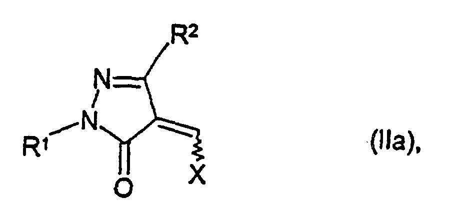 dans laquelle
X représente un groupe amino- ou -O-alkyle (ayant de 1 à 6 atomes de carbone dans le fragment alkyle),
et on fait réagir ces derniers, éventuellement in situ, avec des dérivés d'aniline appropriés de formule 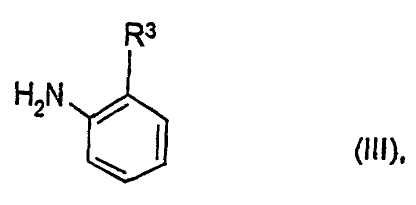 dans laquelle
R³ a les significations données dans les revendications 1 et 2 ou 5,
ou on convertit leurs sels en composés de formule I et/ou **en ce que**, dans le composé de formule I, on transforme un ou plusieurs radicaux en un ou plusieurs autres radicaux, ou on fait réagir un composé de formule II avec un alkylisocyanate en présence d'une base choisie parmi le butyllithium et le méthyllithium.
